# EUROPEAN PATENT SPECIFICATION

(11) **EP 2 004 220 B1**
(45) Date of publication and mention of the grant of the patent: **01.07.2015**
(21) Application number: 07754139.9
(22) Date of filing: 29.03.2007
(51) Int. Cl.: A61K 39/00, A61K 39/40, A61K 39/08, A01N 63/00, A61K 45/00

(54) **METHODS AND COMPOSITIONS FOR VACCINATION OF POULTRY**
VERFAHREN UND ZUSAMMENSETZUNGEN FÜR GEFLÜGELIMPFUNG
PROCÉDÉS ET COMPOSITIONS DESTINÉS À LA VACCINATION DES VOLAILLES

(30) Priority: 30.03.2006 US 787567 P
(43) Date of publication of application: 24.12.2008
(73) Proprietor: EMBREX INC., Research Triangle Park North Carolina 27709-3989 (US)
(72) Inventor: DOELLING, Vivian W., Cary, North Carolina 27511 (US); POSTON, Rebecca M., Raleigh, North Carolina 27615 (US); HEGGEN-PEAY, Cherilyn L., Apex, North Carolina 27502 (US); AVAKIAN, Alan P., Raleigh, North Carolina 27607 (US); TYCZKOWSKI, Julius, Cary, North Carolina 27511 (US)
(74) Representative: Mannion, Sally Kim
(86) International application number: PCT/US2007/007569
(87) International publication number: WO 2007/126816

(56) References cited:
- WO-A2-2004/020585
- US-A- 4 458 630
- US-A- 5 688 513
- US-A- 5 817 320
- US-A1- 2003 044 414
- US-B1- 6 495 146
- MCREYNOLDS ET AL.: 'Evaluation of immunosuppressants and dietary mechanisms in an experimental disease model for necrotic enteritis' POULTRY SCIENCE vol. 83, 2004, pages 1948 - 1952, XP008130885
- THOMPSON ET AL.: 'Live attenuated vaccine-based control of necrotic enteritis of broiler chickens' VETERINARY MICROBIOLOGY vol. 113, 2006, pages 25 - 34, XP005287175
- LOVLAND ET AL.: 'Maternal vaccination against subclinical necrotic enteritis in broilers' AVIAN PATHOLOGY vol. 33, no. 1, 2004, pages 83 - 92, XP009071423

### SEQUENCE LISTING

<110> Embrex, Inc.
   Doelling, Vivian W.
   Poston, Rebecca
   Heggan-Peay, Cherilyn I.
   Avakian, Alan P.
   Tyczkowski, Julius
<120> METHODS AND COMPOSITIONS FOR VACCINATION OF POULTRY
<130> 5175-184WO
<150> US 60/787,567
   <151> 2006-03-30
<160> 13
<170> PatentIn version 3.3
<210> 1
   <211> 1113
   <212> DNA
   <213> Clostridium perfringens
<220>
   <221> CDS
   <222> (1)..(1110)
<400> 1
   tgg gat gga aag att gat gga aca gga act cat gct atg att gta act      48
   Trp Asp Gly Lys Ile Asp Gly Thr Gly Thr His Ala Met Ile Val Thr
   1               5                   10                  15
   caa ggg gtt tca atc tta gaa aat gat ctg tcc aaa aat gaa cca gaa      96
   Gln Gly Val Ser Ile Leu Glu Asn Asp Leu Ser Lys Asn Glu Pro Glu
               20                  25                  30
   agt gta aga aaa aac tta gag att tta aaa gag aac atg cat gag ctt     144
   Ser Val Arg Lys Asn Leu Glu Ile Leu Lys Glu Asn Met His Glu Leu
           35                  40                  45
   caa tta ggt tct act tat cca gat tat gat aag aat gca tat gat cta     192
   Gln Leu Gly Ser Thr Tyr Pro Asp Tyr Asp Lys Asn Ala Tyr Asp Leu
       50                  55                  60
   tat caa gat cat ttc tgg gat cct gat aca gat aat aat ttc tca aag     240
   Tyr Gln Asp His Phe Trp Asp Pro Asp Thr Asp Asn Asn Phe Ser Lys
   65                  70                  75                  80
   gat aat agt tgg tat tta gct tat tct ata cct gac aca ggg gaa tca     288
   Asp Asn Ser Trp Tyr Leu Ala Tyr Ser Ile Pro Asp Thr Gly Glu Ser
                   85                  90                  95
   caa ata aga aaa ttt tca gca tta gct aga tat gaa tgg caa aga gga     336
   Gln Ile Arg Lys Phe Ser Ala Leu Ala Arg Tyr Glu Trp Gln Arg Gly
               100                 105                 110
   aac tat aaa caa gct aca ttc tat ctt gga gag gct atg cac tat ttt     384
   Asn Tyr Lys Gln Ala Thr Phe Tyr Leu Gly Glu Ala Met His Tyr Phe
   115 120 125
   gga gat ata gat act cca tat cat cct gct aat gtt act gcc gtt gat 432
   Gly Asp Ile Asp Thr Pro Tyr His Pro Ala Asn Val Thr Ala Val Asp
   130 135 140
   agc gca gga cat gtt aag ttt gaa act ttt gca gag gaa aga aaa gaa 480
   Ser Ala Gly His Val Lys Phe Glu Thr Phe Ala Glu Glu Arg Lys Glu
   145 150 155 160
   cag tat aaa ata aac aca gca ggt tgc aaa act aat gag gat ttt tat 528
   Gln Tyr Lys Ile Asn Thr Ala Gly Cys Lys Thr Asn Glu Asp Phe Tyr
   165 170 175
   gct gat atc tta aaa aac aag gat ttt aat gca tgg tca aaa gaa tat 576
   Ala Asp Ile Leu Lys Asn Lys Asp Phe Asn Ala Trp Ser Lys Glu Tyr
   180 185 190
   gca aga ggt ttt gct aaa aca gga aaa tca ata tac tat agt cat gct 624
   Ala Arg Gly Phe Ala Lys Thr Gly Lys Ser Ile Tyr Tyr Ser His Ala
   195 200 205
   agc atg agt cat agt tgg gat gat tgg gat tat gca gca aag gta act 672
   Ser Met Ser His Ser Trp Asp Asp Trp Asp Tyr Ala Ala Lys Val Thr
   210 215 220
   tta gct aac tct caa aaa gga aca gca gga tat att tat aga ttc tta 720
   Leu Ala Asn Ser Gln Lys Gly Thr Ala Gly Tyr Ile Tyr Arg Phe Leu
   225 230 235 240
   cac gat gta tca gag ggt aat gat cca tca gtt gga aag aat gta aaa 768
   His Asp Val Ser Glu Gly Asn Asp Pro Ser Val Gly Lys Asn Val Lys
   245 250 255
   gaa cta gta gct tac ata tca act agt ggt gaa aaa gat gct gga aca 816
   Glu Leu Val Ala Tyr Ile Ser Thr Ser Gly Glu Lys Asp Ala Gly Thr
   260 265 270
   gat gac tac atg tat ttt gga atc aaa aca aag gat gga aaa act caa 864
   Asp Asp Tyr Met Tyr Phe Gly Ile Lys Thr Lys Asp Gly Lys Thr Gln
   275 280 285
   gaa tgg gaa atg gac aac cca gga aat gat ttt atg act gga agt aaa 912
   Glu Trp Glu Met Asp Asn Pro Gly Asn Asp Phe Met Thr Gly Ser Lys
   290 295 300
   gac act tat act ttc aaa tta aaa gat gaa aat cta aaa att gat gat 960
   Asp Thr Tyr Thr Phe Lys Leu Lys Asp Glu Asn Leu Lys Ile Asp Asp
   305 310 315 320
   ata caa aat atg tgg att aga aaa aga aaa tat aca gca ttc cca gat 1008
   Ile Gln Asn Met Trp Ile Arg Lys Arg Lys Tyr Thr Ala Phe Pro Asp
   325 330 335
   gct tat aag cca gaa aac ata aag ata ata gca aat gga aaa gtt gta 1056
   Ala Tyr Lys Pro Glu Asn Ile Lys Ile Ile Ala Asn Gly Lys Val Val
   340 345 350
   gta gac aaa gat ata aat gag tgg att tca gga aat tca act tat aat 1104
   Val Asp Lys Asp Ile Asn Glu Trp Ile Ser Gly Asn Ser Thr Tyr Asn
   355 360 365
   ata aaa taa                                                           1113
   Ile Lys
       370
<210> 2
   <211> 370
   <212> PRT
   <213> Clostridium perfringens
<400> 2
   Trp Asp Gly Lys Ile Asp Gly Thr Gly Thr His Ala Met Ile Val Thr
   1               5                   10                  15
   Gln Gly Val Ser Ile Leu Glu Asn Asp Leu Ser Lys Asn Glu Pro Glu
               20                  25                  30
   Ser Val Arg Lys Asn Leu Glu Ile Leu Lys Glu Asn Met His Glu Leu
           35                  40                  45
   Gln Leu Gly Ser Thr Tyr Pro Asp Tyr Asp Lys Asn Ala Tyr Asp Leu
       50                  55                  60
   Tyr Gln Asp His Phe Trp Asp Pro Asp Thr Asp Asn Asn Phe Ser Lys
   65                  70                  75                  80
   Asp Asn Ser Trp Tyr Leu Ala Tyr Ser Ile Pro Asp Thr Gly Glu Ser
                   85                  90                  95
   Gln Ile Arg Lys Phe Ser Ala Leu Ala Arg Tyr Glu Trp Gln Arg Gly
               100                 105                 110
   Asn Tyr Lys Gln Ala Thr Phe Tyr Leu Gly Glu Ala Met His Tyr Phe
           115                 120                 125
   Gly Asp Ile Asp Thr Pro Tyr His Pro Ala Asn Val Thr Ala Val Asp
       130                 135                 140
   Ser Ala Gly His Val Lys Phe Glu Thr Phe Ala Glu Glu Arg Lys Glu
   145                 150                 155                 160
   Gln Tyr Lys Ile Asn Thr Ala Gly Cys Lys Thr Asn Glu Asp Phe Tyr
                   165                 170                 175
   Ala Asp Ile Leu Lys Asn Lys Asp Phe Asn Ala Trp Ser Lys Glu Tyr
               180                 185                 190
   Ala Arg Gly Phe Ala Lys Thr Gly Lys Ser Ile Tyr Tyr Ser His Ala
   195 200 205
   Ser Met Ser His Ser Trp Asp Asp Trp Asp Tyr Ala Ala Lys Val Thr
   210 215 220
   Leu Ala Asn Ser Gln Lys Gly Thr Ala Gly Tyr Ile Tyr Arg Phe Leu
   225 230 235 240
   His Asp Val Ser Glu Gly Asn Asp Pro Ser Val Gly Lys Asn Val Lys
   245 250 255
   Glu Leu Val Ala Tyr Ile Ser Thr Ser Gly Glu Lys Asp Ala Gly Thr
   260 265 270
   Asp Asp Tyr Met Tyr Phe Gly Ile Lys Thr Lys Asp Gly Lys Thr Gln
   275 280 285
   Glu Trp Glu Met Asp Asn Pro Gly Asn Asp Phe Met Thr Gly Ser Lys
   290 295 300
   Asp Thr Tyr Thr Phe Lys Leu Lys Asp Glu Asn Leu Lys Ile Asp Asp
   305 310 315 320
   Ile Gln Asn Met Trp Ile Arg Lys Arg Lys Tyr Thr Ala Phe Pro Asp
   325 330 335
   Ala Tyr Lys Pro Glu Asn Ile Lys Ile Ile Ala Asn Gly Lys Val Val
   340 345 350
   Val Asp Lys Asp Ile Asn Glu Trp Ile Ser Gly Asn Ser Thr Tyr Asn
   355 360 365
   Ile Lys
   370
<210> 3
   <211> 374
   <212> DNA
   <213> Clostridium perfringens
<220>
   <221> CDS
   <222> (1)..(372)
<400> 3
   aat gat cca tca gtt gga aag aat gta aaa gaa cta gta gct tac ata 48
   Asn Asp Pro Ser Val Gly Lys Asn Val Lys Glu Leu Val Ala Tyr Ile
   1               5                   10                  15
   tca act agt ggt gaa aaa gat gct gga aca gat gac tac atg tat ttt       96
   Ser Thr Ser Gly Glu Lys Asp Ala Gly Thr Asp Asp Tyr Met Tyr Phe
               20                  25                  30
   gga atc aaa aca aag gat gga aaa act caa gaa tgg gaa atg gac aac      144
   Gly Ile Lys Thr Lys Asp Gly Lys Thr Gln Glu Trp Glu Met Asp Asn
           35                  40                  45
   cca gga aat gat ttt atg act gga agt aaa gac act tat act ttc aaa      192
   Pro Gly Asn Asp Phe Met Thr Gly Ser Lys Asp Thr Tyr Thr Phe Lys
       50                  55                  60
   tta aaa gat gaa aat cta aaa att gat gat ata caa aat atg tgg att      240
   Leu Lys Asp Glu Asn Leu Lys Ile Asp Asp Ile Gln Asn Met Trp Ile
   65                  70                  75                  80
   aga aaa aga aaa tat aca gca ttc cca gat gct tat aag cca gaa aac      288
   Arg Lys Arg Lys Tyr Thr Ala Phe Pro Asp Ala Tyr Lys Pro Glu Asn
                   85                  90                  95
   ata aag ata ata gca aat gga aaa gtt gta gta gac aaa gat ata aat      336
   Ile Lys Ile Ile Ala Asn Gly Lys Val Val Val Asp Lys Asp Ile Asn
               100                 105                 110
   gag tgg att tca gga aat tca act tat aat ata aaa ta                   374
   Glu Trp Ile Ser Gly Asn Ser Thr Tyr Asn Ile Lys
           115                 120
<210> 4
   <211> 124
   <212> PRT
   <213> Clostridium perfringens
<400> 4
   Asn Asp Pro Ser Val Gly Lys Asn Val Lys Glu Leu Val Ala Tyr Ile
   1               5                   10                  15
   Ser Thr Ser Gly Glu Lys Asp Ala Gly Thr Asp Asp Tyr Met Tyr Phe
               20                  25                  30
   Gly Ile Lys Thr Lys Asp Gly Lys Thr Gln Glu Trp Glu Met Asp Asn
           35                  40                  45
   Pro Gly Asn Asp Phe Met Thr Gly Ser Lys Asp Thr Tyr Thr Phe Lys
       50                  55                  60
   Leu Lys Asp Glu Asn Leu Lys Ile Asp Asp Ile Gln Asn Met Trp Ile
   65                  70                  75                  80
   Arg Lys Arg Lys Tyr Thr Ala Phe Pro Asp Ala Tyr Lys Pro Glu Asn
                   85                  90                  95
   Ile Lys Ile Ile Ala Asn Gly Lys Val Val Val Asp Lys Asp Ile Asn
   100 105 110
   Glu Trp Ile Ser Gly Asn Ser Thr Tyr Asn Ile Lys
   115 120
<210> 5
   <211> 834
   <212> DNA
   <213> Clostridium perfringens
<220>
   <221> CDS
   <222> (1)..(834)
<400> 5
   tgg gat gga aag att gat gga aca gga act cat gct atg att gta act 48
   Trp Asp Gly Lys Ile Asp Gly Thr Gly Thr His Ala Met Ile Val Thr
   1 5 10 15
   caa ggg gtt tca atc tta gaa aat gat ctg tcc aaa aat gaa cca gaa 96
   Gln Gly Val Ser Ile Leu Glu Asn Asp Leu Ser Lys Asn Glu Pro Glu
   20 25 30
   agt gta aga aaa aac tta gag att tta aaa gag aac atg cat gag ctt 144
   Ser Val Arg Lys Asn Leu Glu Ile Leu Lys Glu Asn Met His Glu Leu
   35 40 45
   caa tta ggt tct act tat cca gat tat gat aag aat gca tat gat cta 192
   Gln Leu Gly Ser Thr Tyr Pro Asp Tyr Asp Lys Asn Ala Tyr Asp Leu
   50 55 60
   tat caa gat cat ttc tgg gat cct gat aca gat aat aat ttc tca aag 240
   Tyr Gln Asp His Phe Trp Asp Pro Asp Thr Asp Asn Asn Phe Ser Lys
   65 70 75 80
   gat aat agt tgg tat tta gct tat tct ata cct gac aca ggg gaa tca 288
   Asp Asn Ser Trp Tyr Leu Ala Tyr Ser Ile Pro Asp Thr Gly Glu Ser
   85 90 95
   caa ata aga aaa ttt tca gca tta gct aga tat gaa tgg caa aga gga 336
   Gln Ile Arg Lys Phe Ser Ala Leu Ala Arg Tyr Glu Trp Gln Arg Gly
   100 105 110
   aac tat aaa caa gct aca ttc tat ctt gga gag gct atg cac tat ttt 384
   Asn Tyr Lys Gln Ala Thr Phe Tyr Leu Gly Glu Ala Met His Tyr Phe
   115 120 125
   gga gat ata gat act cca tat cat cct gct aat gtt act gcc gtt gat 432
   Gly Asp Ile Asp Thr Pro Tyr His Pro Ala Asn Val Thr Ala Val Asp
   130 135 140
   agc gca gga cat gtt aag ttt gaa act ttt gca gag gaa aga aaa gaa 480
   Ser Ala Gly His Val Lys Phe Glu Thr Phe Ala Glu Glu Arg Lys Glu
   145 150 155 160
   cag tat aaa ata aac aca gca ggt tgc aaa act aat gag gat ttt tat 528
   Gln Tyr Lys Ile Asn Thr Ala Gly Cys Lys Thr Asn Glu Asp Phe Tyr
   165 170 175
   gct gat atc tta aaa aac aag gat ttt aat gca tgg tca aaa gaa tat 576
   Ala Asp Ile Leu Lys Asn Lys Asp Phe Asn Ala Trp Ser Lys Glu Tyr
   180 185 190
   gca aga ggt ttt gct aaa aca gga aaa tca ata tac tat agt cat gct 624
   Ala Arg Gly Phe Ala Lys Thr Gly Lys Ser Ile Tyr Tyr Ser His Ala
   195 200 205
   agc atg agt cat agt tgg gat gat tgg gat tat gca gca aag gta act 672
   Ser Met Ser His Ser Trp Asp Asp Trp Asp Tyr Ala Ala Lys Val Thr
   210 215 220
   tta gct aac tct caa aaa gga aca gca gga tat att tat aga ttc tta 720
   Leu Ala Asn Ser Gln Lys Gly Thr Ala Gly Tyr Ile Tyr Arg Phe Leu
   225 230 235 240
   cac gat gta tca gag ggt aat gat cca tca gtt gga aag aat gta aaa 768
   His Asp Val Ser Glu Gly Asn Asp Pro Ser Val Gly Lys Asn Val Lys
   245 250 255
   gaa cta gta gct tac ata tca act agt ggt gaa aaa gat gct gga aca 816
   Glu Leu Val Ala Tyr Ile Ser Thr Ser Gly Glu Lys Asp Ala Gly Thr
   260 265 270
   gat gac tac atg tat ttt 834
   Asp Asp Tyr Met Tyr Phe
   275
<210> 6
   <211> 278
   <212> PRT
   <213> Clostridium perfringens
<400> 6
   Trp Asp Gly Lys Ile Asp Gly Thr Gly Thr His Ala Met Ile Val Thr
   1 5 10 15
   Gln Gly Val Ser Ile Leu Glu Asn Asp Leu Ser Lys Asn Glu Pro Glu
   20 25 30
   Ser Val Arg Lys Asn Leu Glu Ile Leu Lys Glu Asn Met His Glu Leu
   35 40 45
   Gln Leu Gly Ser Thr Tyr Pro Asp Tyr Asp Lys Asn Ala Tyr Asp Leu
   50 55 60
   Tyr Gln Asp His Phe Trp Asp Pro Asp Thr Asp Asn Asn Phe Ser Lys
   65 70 75 80
   Asp Asn Ser Trp Tyr Leu Ala Tyr Ser Ile Pro Asp Thr Gly Glu Ser
   85 90 95
   Gln Ile Arg Lys Phe Ser Ala Leu Ala Arg Tyr Glu Trp Gln Arg Gly
   100 105 110
   Asn Tyr Lys Gln Ala Thr Phe Tyr Leu Gly Glu Ala Met His Tyr Phe
   115 120 125
   Gly Asp Ile Asp Thr Pro Tyr His Pro Ala Asn Val Thr Ala Val Asp
   130 135 140
   Ser Ala Gly His Val Lys Phe Glu Thr Phe Ala Glu Glu Arg Lys Glu
   145 150 155 160
   Gln Tyr Lys Ile Asn Thr Ala Gly Cys Lys Thr Asn Glu Asp Phe Tyr
   165 170 175
   Ala Asp Ile Leu Lys Asn Lys Asp Phe Asn Ala Trp Ser Lys Glu Tyr
   180 185 190
   Ala Arg Gly Phe Ala Lys Thr Gly Lys Ser Ile Tyr Tyr Ser His Ala
   195 200 205
   Ser Met Ser His Ser Trp Asp Asp Trp Asp Tyr Ala Ala Lys Val Thr
   210 215 220
   Leu Ala Asn Ser Gln Lys Gly Thr Ala Gly Tyr Ile Tyr Arg Phe Leu
   225 230 235 240
   His Asp Val Ser Glu Gly Asn Asp Pro Ser Val Gly Lys Asn Val Lys
   245 250 255
   Glu Leu Val Ala Tyr Ile Ser Thr Ser Gly Glu Lys Asp Ala Gly Thr
   260 265 270
   Asp Asp Tyr Met Tyr Phe
   275
<210> 7
   <211> 120
   <212> DNA
   <213> Clostridium perfringens
<220>
   <221> CDS
   <222> (1)..(120)
<400> 7
   tac ata tca act agt ggt gaa aaa gat gct gga aca gat gac tac atg        48
   Tyr Ile Ser Thr Ser Gly Glu Lys Asp Ala Gly Thr Asp Asp Tyr Met
   1               5                   10                  15
   tat ttt gga atc aaa aca aag gat gga aaa act caa gaa tgg gaa atg        96
   Tyr Phe Gly Ile Lys Thr Lys Asp Gly Lys Thr Gln Glu Trp Glu Met
               20                  25                  30
   gac aac cca gga aat gat ttt atg                                        120
   Asp Asn Pro Gly Asn Asp Phe Met
           35                  40
<210> 8
   <211> 40
   <212> PRT
   <213> Clostridium perfringens
<400> 8
   Tyr Ile Ser Thr Ser Gly Glu Lys Asp Ala Gly Thr Asp Asp Tyr Met
   1               5                   10                  15
   Tyr Phe Gly Ile Lys Thr Lys Asp Gly Lys Thr Gln Glu Trp Glu Met
               20                  25                  30
   Asp Asn Pro Gly Asn Asp Phe Met
           35                  40
<210> 9
   <211> 1197
   <212> DNA
   <213> Clostridium perfringens
<220>
   <221> CDS
   <222> (1)..(1194)
<400> 9
   atg aaa aga aag att tgt aag gcg ctt att tgt gct acg cta gca act        48
   Met Lys Arg Lys Ile Cys Lys Ala Leu Ile Cys Ala Thr Leu Ala Thr
   1               5                   10                  15
   agc cta tgg gct ggg gca tca act aaa gtc tac gct tgg gat gga aag        96
   Ser Leu Trp Ala Gly Ala Ser Thr Lys Val Tyr Ala Trp Asp Gly Lys
               20                  25                  30
   att gat gga aca gga act cat gct atg att gta act caa ggg gtt tca        144
   Ile Asp Gly Thr Gly Thr His Ala Met Ile Val Thr Gln Gly Val Ser
           35                  40                  45
   atc tta gaa aat gat ctg tcc aaa aat gaa cca gaa agt gta aga aaa        192
   Ile Leu Glu Asn Asp Leu Ser Lys Asn Glu Pro Glu Ser Val Arg Lys
       50                  55                  60
   aac tta gag att tta aaa gag aac atg cat gag ctt caa tta ggt tct        240
   Asn Leu Glu Ile Leu Lys Glu Asn Met His Glu Leu Gln Leu Gly Ser
   65                  70                  75                  80
   act tat cca gat tat gat aag aat gca tat gat cta tat caa gat cat      288
   Thr Tyr Pro Asp Tyr Asp Lys Asn Ala Tyr Asp Leu Tyr Gln Asp His
                   85                  90                  95
   ttc tgg gat cct gat aca gat aat aat ttc tca aag gat aat agt tgg      336
   Phe Trp Asp Pro Asp Thr Asp Asn Asn Phe Ser Lys Asp Asn Ser Trp
               100                 105                 110
   tat tta gct tat tct ata cct gac aca ggg gaa tca caa ata aga aaa      384
   Tyr Leu Ala Tyr Ser Ile Pro Asp Thr Gly Glu Ser Gln Ile Arg Lys
           115                 120                 125
   ttt tca gca tta gct aga tat gaa tgg caa aga gga aac tat aaa caa      432
   Phe Ser Ala Leu Ala Arg Tyr Glu Trp Gln Arg Gly Asn Tyr Lys Gln
       130                 135                 140
   gct aca ttc tat ctt gga gag gct atg cac tat ttt gga gat ata gat      480
   Ala Thr Phe Tyr Leu Gly Glu Ala Met His Tyr Phe Gly Asp Ile Asp
   145                 150                 155                 160
   act cca tat cat cct gct aat gtt act gcc gtt gat agc gca gga cat      528
   Thr Pro Tyr His Pro Ala Asn Val Thr Ala Val Asp Ser Ala Gly His
                   165                 170                 175
   gtt aag ttt gaa act ttt gca gag gaa aga aaa gaa cag tat aaa ata      576
   Val Lys Phe Glu Thr Phe Ala Glu Glu Arg Lys Glu Gln Tyr Lys Ile
               180                 185                 190
   aac aca gca ggt tgc aaa act aat gag gat ttt tat gct gat atc tta      624
   Asn Thr Ala Gly Cys Lys Thr Asn Glu Asp Phe Tyr Ala Asp Ile Leu
           195                 200                 205
   aaa aac aag gat ttt aat gca tgg tca aaa gaa tat gca aga ggt ttt      672
   Lys Asn Lys Asp Phe Asn Ala Trp Ser Lys Glu Tyr Ala Arg Gly Phe
       210                 215                 220
   gct aaa aca gga aaa tca ata tac tat agt cat gct agc atg agt cat      720
   Ala Lys Thr Gly Lys Ser Ile Tyr Tyr Ser His Ala Ser Met Ser His
   225                 230                 235                 240
   agt tgg gat gat tgg gat tat gca gca aag gta act tta gct aac tct      768
   Ser Trp Asp Asp Trp Asp Tyr Ala Ala Lys Val Thr Leu Ala Asn Ser
                   245                 250                 255
   caa aaa gga aca gca gga tat att tat aga ttc tta cac gat gta tca      816
   Gln Lys Gly Thr Ala Gly Tyr Ile Tyr Arg Phe Leu His Asp Val Ser
               260                 265                 270
   gag ggt aat gat cca tca gtt gga aag aat gta aaa gaa cta gta gct      864
   Glu Gly Asn Asp Pro Ser Val Gly Lys Asn Val Lys Glu Leu Val Ala
           275                 280                 285
   tac ata tca act agt ggt gaa aaa gat gct gga aca gat gac tac atg      912
   Tyr Ile Ser Thr Ser Gly Glu Lys Asp Ala Gly Thr Asp Asp Tyr Met
       290                 295                 300
   tat ttt gga atc aaa aca aag gat gga aaa act caa gaa tgg gaa atg      960
   Tyr Phe Gly Ile Lys Thr Lys Asp Gly Lys Thr Gln Glu Trp Glu Met
   305                 310                 315                 320
   gac aac cca gga aat gat ttt atg act gga agt aaa gac act tat act      1008
   Asp Asn Pro Gly Asn Asp Phe Met Thr Gly Ser Lys Asp Thr Tyr Thr
                   325                 330                 335
   ttc aaa tta aaa gat gaa aat cta aaa att gat gat ata caa aat atg      1056
   Phe Lys Leu Lys Asp Glu Asn Leu Lys Ile Asp Asp Ile Gln Asn Met
               340                 345                 350
   tgg att aga aaa aga aaa tat aca gca ttc cca gat gct tat aag cca      1104
   Trp Ile Arg Lys Arg Lys Tyr Thr Ala Phe Pro Asp Ala Tyr Lys Pro
           355                 360                 365
   gaa aac ata aag ata ata gca aat gga aaa gtt gta gta gac aaa gat      1152
   Glu Asn Ile Lys Ile Ile Ala Asn Gly Lys Val Val Val Asp Lys Asp
       370                 375                 380
   ata aat gag tgg att tca gga aat tca act tat aat ata aaa taa          1197
   Ile Asn Glu Trp Ile Ser Gly Asn Ser Thr Tyr Asn Ile Lys
   385                 390                 395
<210> 10
   <211> 398
   <212> PRT
   <213> Clostridium perfringens
<400> 10
   Met Lys Arg Lys Ile Cys Lys Ala Leu Ile Cys Ala Thr Leu Ala Thr
   1               5                   10                  15
   Ser Leu Trp Ala Gly Ala Ser Thr Lys Val Tyr Ala Trp Asp Gly Lys
               20                  25                  30
   Ile Asp Gly Thr Gly Thr His Ala Met Ile Val Thr Gln Gly Val Ser
           35                  40                  45
   Ile Leu Glu Asn Asp Leu Ser Lys Asn Glu Pro Glu Ser Val Arg Lys
       50                  55                  60
   Asn Leu Glu Ile Leu Lys Glu Asn Met His Glu Leu Gln Leu Gly Ser
   65                  70                  75                  80
   Thr Tyr Pro Asp Tyr Asp Lys Asn Ala Tyr Asp Leu Tyr Gln Asp His
                   85                  90                  95
   Phe Trp Asp Pro Asp Thr Asp Asn Asn Phe Ser Lys Asp Asn Ser Trp
               100                 105                 110
   Tyr Leu Ala Tyr Ser Ile Pro Asp Thr Gly Glu Ser Gln Ile Arg Lys
           115                 120                 125
   Phe Ser Ala Leu Ala Arg Tyr Glu Trp Gln Arg Gly Asn Tyr Lys Gln
       130                 135                 140
   Ala Thr Phe Tyr Leu Gly Glu Ala Met His Tyr Phe Gly Asp Ile Asp
   145                 150                 155                 160
   Thr Pro Tyr His Pro Ala Asn Val Thr Ala Val Asp Ser Ala Gly His
                   165                 170                 175
   Val Lys Phe Glu Thr Phe Ala Glu Glu Arg Lys Glu Gln Tyr Lys Ile
               180                 185                 190
   Asn Thr Ala Gly Cys Lys Thr Asn Glu Asp Phe Tyr Ala Asp Ile Leu
           195                 200                 205
   Lys Asn Lys Asp Phe Asn Ala Trp Ser Lys Glu Tyr Ala Arg Gly Phe
       210                 215                 220
   Ala Lys Thr Gly Lys Ser Ile Tyr Tyr Ser His Ala Ser Met Ser His
   225                 230                 235                 240
   Ser Trp Asp Asp Trp Asp Tyr Ala Ala Lys Val Thr Leu Ala Asn Ser
                   245                 250                 255
   Gln Lys Gly Thr Ala Gly Tyr Ile Tyr Arg Phe Leu His Asp Val Ser
               260                 265                 270
   Glu Gly Asn Asp Pro Ser Val Gly Lys Asn Val Lys Glu Leu Val Ala
           275                 280                 285
   Tyr Ile Ser Thr Ser Gly Glu Lys Asp Ala Gly Thr Asp Asp Tyr Met
       290                 295                 300
   Tyr Phe Gly Ile Lys Thr Lys Asp Gly Lys Thr Gln Glu Trp Glu Met
   305                 310                 315                 320
   Asp Asn Pro Gly Asn Asp Phe Met Thr Gly Ser Lys Asp Thr Tyr Thr
                   325                 330                 335
   Phe Lys Leu Lys Asp Glu Asn Leu Lys Ile Asp Asp Ile Gln Asn Met
               340                 345                 350
   Trp Ile Arg Lys Arg Lys Tyr Thr Ala Phe Pro Asp Ala Tyr Lys Pro
           355                 360                 365
   Glu Asn Ile Lys Ile Ile Ala Asn Gly Lys Val Val Val Asp Lys Asp
   370 375 380
   Ile Asn Glu Trp Ile Ser Gly Asn Ser Thr Tyr Asn Ile Lys
   385 390 395
<210> 11
   <211> 336
   <212> PRT
   <213> Clostridium perfringens
<400> 11
   Met Lys Lys Lys Phe Ile Ser Leu Val Ile Val Ser Ser Leu Leu Asn
   1 5 10 15
   Gly Cys Leu Leu Ser Pro Arg Leu Val Tyr Ala Asn Asp Ile Gly Lys
   20 25 30
   Thr Thr Thr Ile Thr Arg Asn Lys Thr Ser Asp Gly Tyr Thr Ile Ile
   35 40 45
   Thr Gln Asn Asp Lys Trp Ile Ile Ser Tyr Gln Ser Val Asp Ser Ser
   50 55 60
   Ser Lys Asn Glu Asp Gly Phe Thr Ala Ser Ile Asp Ala Arg Phe Ile
   65 70 75 80
   Asp Asp Lys Tyr Ser Ser Glu Met Thr Thr Leu Ile Asn Leu Thr Gly
   85 90 95
   Phe Met Ser Ser Lys Lys Glu Asp Val Ile Lys Lys Tyr Asn Leu His
   100 105 110
   Asp Asn Thr Asn Ser Thr Ala Ile Asn Phe Pro Val Arg Tyr Ser Ile
   115 120 125
   Ser Ile Leu Asn Glu Ser Ile Asn Glu Asn Val Lys Ile Val Asp Ser
   130 135 140
   Ile Pro Lys Asn Thr Ile Ser Gln Lys Thr Val Ser Asn Thr Met Gly
   145 150 155 160
   Tyr Lys Ile Gly Gly Ser Ile Glu Ile Glu Glu Asn Lys Pro Lys Ala
   165 170 175
   Ser Ile Glu Ser Glu Tyr Ala Glu Ser Ser Thr Ile Glu Tyr Val Gln
   180 185 190
   Pro Asp Phe Ser Thr Ile Gln Thr Asp His Ser Thr Ser Lys Ala Ser
   195 200 205
   Trp Asp Thr Lys Phe Thr Glu Thr Thr Arg Gly Asn Tyr Asn Leu Lys
   210 215 220
   Ser Asn Asn Pro Val Tyr Gly Asn Glu Met Phe Met Tyr Gly Arg Tyr
   225 230 235 240
   Thr Asn Val Pro Ala Thr Glu Asn Ile Ile Pro Asp Tyr Gln Met Ser
   245 250 255
   Lys Leu Ile Thr Gly Gly Leu Asn Pro Asn Met Ser Val Val Leu Thr
   260 265 270
   Ala Pro Asn Gly Thr Glu Glu Ser Ile Ile Lys Val Lys Met Glu Arg
   275 280 285
   Glu Arg Asn Cys Tyr Tyr Leu Asn Trp Asn Gly Ala Asn Trp Val Gly
   290 295 300
   Gln Val Tyr Ser Arg Leu Ala Phe Asp Thr Pro Asn Val Asp Ser His
   305 310 315 320
   Ile Phe Thr Phe Lys Ile Asn Trp Leu Thr His Lys Val Thr Ala Ile
   325 330 335
<210> 12
   <211> 328
   <212> PRT
   <213> Clostridium perfringens
<400> 12
   Met Lys Lys Asn Leu Val Lys Ser Leu Ala Ile Ala Ser Ala Val Ile
   1 5 10 15
   Ser Ile Tyr Ser Ile Val Asn Ile Val Ser Pro Thr Asn Val Ile Ala
   20 25 30
   Lys Glu Ile Ser Asn Thr Val Ser Asn Glu Met Ser Lys Lys Ala Ser
   35 40 45
   Tyr Asp Asn Val Asp Thr Leu Ile Glu Lys Gly Arg Tyr Asn Thr Lys
   50 55 60
   Tyr Asn Tyr Leu Lys Arg Met Glu Lys Tyr Tyr Pro Asn Ala Met Ala
   65 70 75 80
   Tyr Phe Asp Lys Val Thr Ile Asn Pro Gln Gly Asn Asp Phe Tyr Ile
   85 90 95
   Asn Asn Pro Lys Val Glu Leu Asp Gly Glu Pro Ser Met Asn Tyr Leu
   100 105 110
   Glu Asp Val Tyr Val Gly Lys Ala Leu Leu Thr Asn Asp Thr Gln Gln
   115 120 125
   Glu Gln Lys Leu Lys Ser Gln Ser Phe Thr Cys Lys Asn Thr Asp Thr
   130 135 140
   Val Thr Ala Thr Thr Thr His Thr Val Gly Thr Ser Ile Gln Ala Thr
   145 150 155 160
   Ala Lys Phe Thr Val Pro Phe Asn Glu Thr Gly Val Ser Leu Thr Thr
   165 170 175
   Ser Tyr Ser Phe Ala Asn Thr Asn Thr Asn Thr Asn Ser Lys Glu Ile
   180 185 190
   Thr His Asn Val Pro Ser Gln Asp Ile Leu Val Pro Ala Asn Thr Thr
   195 200 205
   Val Glu Val Ile Ala Tyr Leu Lys Lys Val Asn Val Lys Gly Asn Val
   210 215 220
   Lys Leu Val Gly Gln Val Ser Gly Ser Glu Trp Gly Glu Ile Pro Ser
   225 230 235 240
   Tyr Leu Ala Phe Pro Arg Asp Gly Tyr Lys Phe Ser Leu Ser Asp Thr
   245 250 255
   Val Asn Lys Ser Asp Leu Asn Glu Asp Gly Thr Ile Asn Ile Asn Gly
   260 265 270
   Lys Gly Asn Tyr Ser Ala Val Met Gly Asp Glu Leu Ile Val Lys Val
   275 280 285
   Arg Asn Leu Asn Thr Asn Asn Val Gln Glu Tyr Val Ile Pro Val Asp
   290 295 300
   Lys Lys Glu Lys Ser Asn Asp Ser Asn Ile Val Lys Tyr Arg Ser Leu
   305 310 315 320
   Tyr Ile Lys Ala Pro Gly Ile Lys
   325
<210> 13
   <211> 283
   <212> PRT
   <213> Clostridium perfringens
<400> 13
   Lys Ala Ser Tyr Asp Asn Val Asp Thr Leu Ile Glu Lys Gly Arg Tyr
   1 5 10 15
   Asn Thr Lys Tyr Asn Tyr Leu Lys Arg Met Glu Lys Tyr Tyr Pro Asn
   20 25 30
   Ala Met Ala Tyr Phe Asp Lys Val Thr Ile Asn Pro Gln Gly Asn Asp
   35 40 45
   Phe Tyr Ile Asn Asn Pro Lys Val Glu Leu Asp Gly Glu Pro Ser Met
   50 55 60
   Asn Tyr Leu Glu Asp Val Tyr Val Gly Lys Ala Leu Leu Thr Asn Asp
   65 70 75 80
   Thr Gln Gln Glu Gln Lys Leu Lys Ser Gln Ser Phe Thr Cys Lys Asn
   85 90 95
   Thr Asp Thr Val Thr Ala Thr Thr Thr His Thr Val Gly Thr Ser Ile
   100 105 110
   Gln Ala Thr Ala Lys Phe Thr Val Pro Phe Asn Glu Thr Gly Val Ser
   115 120 125
   Leu Thr Thr Ser Tyr Ser Phe Ala Asn Thr Asn Thr Asn Thr Asn Ser
   130 135 140
   Lys Glu Ile Thr His Asn Val Pro Ser Gln Asp Ile Leu Val Pro Ala
   145 150 155 160
   Asn Thr Thr Val Glu Val Ile Ala Tyr Leu Lys Lys Val Asn Val Lys
   165 170 175
   Gly Asn Val Lys Leu Val Gly Gln Val Ser Gly Ser Glu Trp Gly Glu
   180 185 190
   Ile Pro Ser Tyr Leu Ala Phe Pro Arg Asp Gly Tyr Lys Phe Ser Leu
           195                 200                 205
   Ser Asp Thr Val Asn Lys Ser Asp Leu Asn Glu Asp Gly Thr Ile Asn
       210                 215                 220
   Ile Asn Gly Lys Gly Asn Tyr Ser Ala Val Met Gly Asp Glu Leu Ile
   225                 230                 235                     240
   Val Lys Val Arg Asn Leu Asn Thr Asn Asn Val Gln Glu Tyr Val Ile
                   245                 250                 255
   Pro Val Asp Lys Lys Glu Lys Ser Asn Asp Ser Asn Ile Val Lys Tyr
               260                 265                 270
   Arg Ser Leu Tyr Ile Lys Ala Pro Gly Ile Lys
           275                 280

## Description

### FIELD OF THE INVENTION

The present invention provides immunogenic compositions for use in producing an immune response to *Clostridium* species in avian subjects. The immunogenic compositions also protect avian subjects from *Clostridium* infection, and protect avian subjects from related disorders such as necrotic enteritis.

### BACKGROUND OF THE INVENTION

*In ovo* vaccination provides several advantages to the poultry industry over current control methods and potential post-hatch vaccination of birds, including the potential for uniform, automated delivery; co-administration with other vaccines *in ovo,* thereby reducing bird handling post-hatch; and decreased used of antibiotics.

The discovery that certain vaccines (e.g., inactivated or nonreplicating vaccines) can be delivered to the embryo body to elicit a strong immune response (similar or better to that expected when vaccinating birds at day of hatch) allows for the development of automated devices that can target the embryo body (specifically avoiding the amniotic fluid surrounding the embryo body) during *in ovo* application of such vaccines. In addition, knowing that inactivated vaccines should preferentially be delivered to the embryo body allows one to develop vaccine compositions that are more compatible with injection into the body of the embryo. Prior to this discovery it would not have been known or expected that inactivated vaccine would need to be administered preferentially to the embryo body to elicit a strong immune response.

Thus, the present invention is an improvement over the art by providing a more efficient way to administer inactivated (killed) vaccines *in ovo.* Prior to this invention there was no indication that administering inactivated vaccine to the embryonic fluids surrounding the embryo body (amniotic fluid) was any different from administering inactivated vaccine to the embryo body. The present invention demonstrates that inactivated *Clostridium* toxin (toxoid) and/or inactivated *Clostridium* bacterium (bacterin) vaccines need to be delivered *in ovo* to the embryo body proper rather than to the fluids surrounding the embryo body. Knowing that the embryo body is an appropriate target for optimal efficacy allows for the development of inactivated vaccines and delivery methods for the *in ovo* route. The invention encompasses administration of immunogenic compositions for use in inducing an immune response against a *Clostridium* species by *in ovo* injection directly into the embryo body of poultry and other avian species.

Accordingly, there is a need in the art for improved immunogenic compositions for administration *in ovo* to the embryo and methods for inducing an immune response in birds, for protecting birds from infection and/or contamination by avian and other pathogens, and for protecting birds from related disorders.

*Clostridium perfringens* is associated with several diseases in poultry, most notably necrotic enteritis (*C*. *perfringens* type A and type C), but also including cholangiohepatitis, cellulitis, gizzard erosions, and navel infections. While these bacteria represent a component of the normal gut flora, various factors can predispose birds to disease development. Such factors include a diet having high levels of fishmeal, wheat, barley or rye; a high moisture litter; or exposure to Coccidia. Clinically, signs of disease generally include diarrhea, decreased appetite, intestinal lesions, depression, and mortality. Traditionally, these diseases are controlled by antibiotics in the feed; however, overuse of antibiotics can lead to the development of antibiotic-resistant strains of bacteria, which present a significant health risk to humans and animals. Further, in certain jurisdictions, the use of antibiotics is highly disfavored or even prohibited.

Other methods for controlling *Clostridium* include a diet that avoids ingredients that irritate the intestinal mucosal, *e*.*g*., a corn-soy ration; a low moisture litter having absorbent material such as wood shavings or rice hulls; the use of a competitive exclusion product to maintain a healthy balance of intestinal microflora, *e.g.,* Primalac (Star-Labs/Forage Research, Inc., Clarksdale, MO), AVIGUARD™ (Bayer Corporation, Kansas City, MO), and BIO-MOS® (Alltech, Inc., Nicholasville, KY); and intense preventative water acidification or disinfection to minimize losses during a disease period. Vaccination has been used to control Clostridial diseases in other species, including cattle, sheep, goats and swine. Two main types of vaccines to *C*. *perfringens* have been developed for non-poultry species: toxoids (inactivated toxins) and bacterin-toxoids (inactivated ["killed"] bacterial cultures and inactivated toxins). Antitoxins (antibodies specific for the toxin[s]) are also used as prevention against and treatment for Clostridial diseases in non-poultry species.

As far as the inventors are aware, there are no previous reports describing *in ovo* administration (*i*.*e*., to an egg containing a developing avian embryo) of a *C*. *perfringens* vaccine. WO2004/020585 generally discloses *in ovo* administration using *Escherichia coli* heat labile toxin. The use of existing toxoid or toxoid-bacterin vaccines *in ovo* is uncertain because such vaccines generally require the use of adjuvants, which may be harmful to the embryo, may inactivate live vaccines against other organisms that are typically administered during the *in ovo* period (*e*.*g*., to vaccinate against Marek's disease), and/or may be incompatible with existing *in ovo* injection equipment. *In ovo* vaccination against *C*. *perfringens* provides several advantages to the poultry industry over current control methods and potential post-hatch vaccination of birds, including the potential for uniform, automated delivery; co-administration with other vaccines *in ovo,* thereby reducing bird handling post-hatch; and decreased used of antibiotics.

Accordingly, there is a need in the art for improved immunogenic compositions and methods for inducing an immune response against *Clostridium* in birds, for protecting birds from *Clostridium* infection, and for protecting birds from related disorders such as necrotic enteritis.

### SUMMARY OF THE INVENTION

The present invention provides an effective immunizing dose of an immunogenic composition for use in inducing an immune response against *Clostridium* species (e.g., *Clostridium perfringens)* wherein the immunogenic composition comprises an inactivated *Clostridium* toxin (toxoid) and/or an inactivated *Clostridium* bacterium (bacterin), and wherein said composition is administered by *in ovo* injection directly into the embryo body. The immunogenic compositions are for use in birds, for protecting birds from *Clostridium* infection, and/or for protecting birds from related disorders such as necrotic enteritis. The methods of administration can be practiced *in ovo* and/or post-hatch.

As described herein, an avian subject (*e.g.,* a chicken) is immunized against necrotic enteritis by administering *in ovo* (*e.g.,* during the final quarter of incubation) an effective immunizing dose of an immunogenic composition that induces an immune response against *Clostridium perfringens,* wherein the immunogenic composition is administered by *in ovo* injection. The immunogenic composition is administered to the embryo. The immunogenic composition of the present invention can optionally be practiced in combination with other immunization regimens (*e*.*g*., vaccination against infectious bursal disease, Marek's disease, Newcastle disease and/or coccidiosis) and/or *in ovo* feeding of a nutrient formulation and/or enteric modulator.

As described herein, an immunogenic composition comprising an effective immunizing dose of an attenuated *Clostridium* species may be administered in a pharmaceutically acceptable carrier. In certain embodiments, the immunogenic composition further comprises an adjuvant, which can be, for example, a depot adjuvant. Representative adjuvants of this invention include but are not limited to an aluminum salt such as aluminum hydroxide gel (alum), aluminum phosphate, or algannmulin, and/or may also be a salt or mineral gels of calcium, magnesium, iron and/or zinc, and/or may be an insoluble suspension of acylated tyrosine, or acylated sugars, cationically or anionically derivatized polysaccharides, or polyphosphazenes, and/or saponins such as Quil-A, and/or oil emulsions, such as water-in-oil and water-in-oil-in-water and/or complete or incomplete Freund's or any combination thereof. In representative embodiments, the immunogenic composition comprises a water-in-oil-in-water emulsion. Optionally, the immunogenic composition can further comprise one or more additional agents that induce an immune response against other avian pathogens (*e*.*g*., an agent that induces an immune response against *Eimeria,* infectious bursal disease virus, Marek's disease virus and/or Newcastle disease virus) and/or a nutrient formulation and/or an enteric modulator. The one or more additional agents can be immunizing agents that produce a protective immune response against *Eimeria,* infectious bursal disease virus, Marek's disease virus and/or Newcastle disease virus.

Further provided herein is an immunogenic composition comprising in a pharmaceutically acceptable carrier:
(a) an effective immunizing dose of a *Clostridium* toxoid, a *Clostridium* bacterin, a *Clostridium* toxin or any combination of the foregoing; and
(b) an effective immunizing dose of a coccidiosis vaccine, a Marek's disease vaccine, an infectious bursal disease vaccine, a Newcastle disease vaccine, a fowl pox vaccine, or any combination of the foregoing.

Also provided herein is an immunogenic composition comprising in a pharmaceutically acceptable carrier:
(a) an effective immunizing dose of a *Clostridium* toxoid, a *Clostridium* bacterin, a *Clostridium* toxin or any combination of the foregoing; and
(b) an oil emulsion.

As still another aspect, there is provided an immunogenic composition comprising in a pharmaceutically acceptable carrier:
(a) an effective immunizing dose of a *Clostridium* toxoid, a *Clostridium* bacterin, a *Clostridium* toxin or any combination of the foregoing; and
(b) an adjuvant comprising an aluminum derived adjuvant, a saponin, an oil, or any combination of the foregoing.

An avian subject may be immunized against infection by a *Clostridium* species, by administering to the avian subject an effective immunizing dose of a *Clostridium* bacterin-toxoid composition by *in ovo* injection during the final quarter of incubation. In some embodiments of the invention, the species of *Clostridium* can be *Clostridium perfringens.*

An avian subject may be immunized against infection by a *Clostridium* species, by administering to the avian subject an effective immunizing dose of a recombinant toxin or immunogenic fragment thereof of a *Clostridium* species by *in ovo* injection during the final quarter of incubation. In some embodiments, the toxin or immunogenic fragment thereof is a *Clostridium perfringens* toxin or immunogenic fragment thereof.

These and other aspects of the invention are set forth in more detail in the description of the invention below.

### DETAILED DESCRIPTION OF THE INVENTION

Certain aspects of the present invention are based on the unexpected discovery that certain antigenic or immunogenic compositions allow for a more effective immune response in birds when the composition is administered *in ovo* directly to the embryo body.

Thus, in one embodiment, the present invention provides an effective immunizing dose of an immunogenic composition for use in inducing an immune response in an avian subject against a *Clostridium* species, comprising administering the immunogenic composition by *in ovo* injection directly into the embryo body. The composition can be administered directly into skeletal muscle tissue in the embryo and the skeletal muscle tissue can be, but is not limited to, breast muscle tissue and pipping muscle tissue, or the composition can be administered directly into the embryo into the head, neck, shoulder, wing, back, breast, leg or any combination thereof.

Further, the composition can be administered subcutaneous in the embryo body, or the composition can be administered subcutaneous into the head, neck, shoulder, wing, back, breast, leg or any combination thereof.

Any suitable route of administration into the embryo is suitable in the use of the immunogenic composition of this invention. For example, the composition can be administered to the embryo subcutaneously, intradermally, intravenously, intramuscularly, intra-abdominally or any combination thereof.

The avian subject can be any avian and can be a chicken, turkey, duck, goose, pheasant, quail, partridge, guinea, ostrich, emu or peafowl, as well as any other commercially processed avian and/or any avian, the eggs of which are accessible for handling in the methods of this invention

In embodiments wherein the subject is a chicken, it is desirable to administer the composition of this invention *in ovo* during the period from day 15 through day 20 of incubation, and in particular embodiments, the composition can be administered on day 18 or day 19 of incubation. When the subject is a turkey, the composition of this invention can be administered during the period from day 21 through day 28 of incubation and in particular, the compositions can be administered on day 24 or day 25 of incubation. When the subject is a goose, the composition of this invention can be administered during the period from day 23 through day 31 of incubation and in particular, the compositions can be administered on day 28 or day 29 of incubation. When the subject is a duck, the composition of this invention can be administered during the period from day 21 through day 28 of incubation and in particular, the compositions can be administered on day 25 or day 26 of incubation.

For other avian species, the final quarter of incubation and thus the optimal range of days for *in ovo* administration of a composition of this invention can be determined according to methods well known in the art. For example, a muscovy duck has an incubation period in the range of 33-35 days, a ringneck pheasant has an incubation period of 23-24 days, a Japanese quail has an incubation period of 17-18 days, a bobwhite quail has an incubation period of 23 days, a chuckar partridge has an incubation period of 22-23 days, a guinea has an incubation period of 26-28 days and a peafowl has an incubation period of 28 days.

In particular embodiments of this invention, the composition can comprise, consist essentially of and/or consist of an immunogenic composition and an adjuvant. Examples of adjuvants of this invention include an aluminum derived adjuvant, a saponin, mineral gels, polyanions, pluronic polyols, saponin derivatives, lysolecithin and other similar surface active substances, glycosides, all types of oils and any combination thereof. In particular embodiments of this invention, the composition can comprise a water-in-oil-in-water emulsion.

As contemplated herein, in some embodiments of the present invention, the composition of this invention can comprise an adjuvant, which in particular embodiments, can be an adjuvant such as an aluminum derived adjuvant (*e*.*g*., aluminum hydroxide), a saponin (*e*.*g*., Quil-A including QuilA QS21), or an oil (such as Complete or Incomplete Freund's adjuvant), in any combination.

Further examples of an adjuvant of this invention include mineral salts (e.g., aluminum hydroxide; aluminum phosphate; calcium phosphate), oil emulsions and surfactant based formulations (e.g., Freund's emulsified oil adjuvants; Arlacel A; mineral oil; emulsified peanut oil adjuvant (adjuvant 65); MF59 (microfluidized detergent stabilized oil-in-water emulsion); QS21 (purified saponin); AS02 [SBAS2] (oil-in-water + MPL + QS21); Montanide ISA-51; ISA-720 (stabilized water-in-oil emulsion), bacterial products and derivatives [e.g., *Bordatella pertussis; Corynebacterium granulosum* derived P40 component; lipopolysaccharide (adjuvant for both humoral and cell-mediated immunity); *Mycobacterium* and its components (MDPs, not acceptable adjuvants in humans); cholera toxin (mucosal adjuvant)], microbial derivatives (natural and synthetic) [e.g., monophosphoryl lipid A (MPL); Detox (MPL + *M*. *phlei* cell wall skeleton); AGP [RC-529] (synthetic acylated monosaccharide); DC-Chol (lipoidal immunostimulators able to self-organize into liposomes); OM-174 (lipid A derivative); CpG motifs (synthetic oligonucleotides containing immunostimulatory CpG motifs); modified LT and CT (genetically modified bacterial toxins to provide non-toxic adjuvant effects)], endogenous chicken immunomodulators [cytokines; antibodies; hGM-CSF or hIL-12 (cytokines that can be administered either as protein or plasmid encoded); Immudaptin (C3d tandem array); Squalene], particulate adjuvants [virosomes (unilamellar liposomal vehicles incorporating antigen); AS04 ([SBAS4] Al salt with MPL); ISCOMs (structured complex of saponins and lipids); polylactide co-glycolide (PLG), and inert vehicles (gold particles; silver particles).

Additional adjuvants of this invention can include mineral gels, polyanions, pluronic polyols, saponin derivatives, lysolecithin and other similar surface active substances. Further adjuvants can include toll-like receptor (TLR) agonists, including, for example, agonists of TLR-1 (e.g. tri-acyl lipopeptides); agonists of TLR-2 [e.g. peptidoglycan of gram-positive bacteria like *streptococci* and staphylococci; lipoteichoic acid]; agonists of TLR-3 (e.g. double-stranded RNA and their analogs such as poly 1 :C); agonists of TLR-4 [e.g. lipopolysaccharide (endotoxin) of gram-negative bacteria like *Salmonella* and *E*. *coli*]; agonists of TLR-5 (e.g. flagellin of motile bacteria like *Listeria*); agonists of TLR-6 [e.g. with TLR-2 peptidoglycan and certain lipids (diacyl lipopeptides)]; agonists of TLR-7 [e.g. single-stranded RNA (ssRNA) genomes of such viruses as influenza, measles, and mumps; and small synthetic guanosine- base antiviral molecules like loxoribine and ssRNA and their analogs]; agonists of TLR-8 (e.g. binds ssRNA); agonists of TLR-9 (e.g. unmethylated CpG of the DNA of the pathogen and their analogs; agonists of TLR-10 (function not defined) and TLR-11- (e.g. binds proteins expressed by several infectious protozoans (Apicomplexa). Chickens have a well developed TLR system with approximately 10 TLRs broadly similar to those detected in mammals.

More examples of adjuvants of this invention include complement receptors (secreted PRRs), wherein C3d (complement component is activated by microbial CHO. The complement pathway leads to opsonization of the pathogen and quick phagocytosis.

An adjuvant of this invention also can be an amino acid sequence that is a peptide, a protein fragment or a whole protein that functions as the adjuvant, or the adjuvant can be a nucleic acid encoding a peptide, protein fragment or whole protein that functions as an adjuvant. As used herein, "adjuvant" describes a substance, which can be any immunomodulating substance capable of being combined with the polypeptide or nucleic acid vaccine to enhance, improve or otherwise modulate an immune response in a subject without deleterious effect on the subject.

An adjuvant of this invention can be, but is not limited to, for example, an immunostimulatory cytokine (including, but not limited to, GM/CSF, interleukin-2, interleukin-12, interferon-gamma, interleukin-4, tumor necrosis factor-alpha, interleukin-1, hematopoietic factor flt3L, CD40L, B7.1 co-stimulatory molecules and B7.2 co-stimulatory molecules), SYNTEX adjuvant formulation 1 (SAF-1) composed of 5 percent (wt/vol) squalene (DASF, Parsippany, N.J.), 2.5 percent Pluronic, L121 polymer (Aldrich Chemical, Milwaukee), and 0.2 percent polysorbate (Tween 80, Sigma) in phosphate-buffered saline. Suitable adjuvants also include an aluminum salt such as aluminum hydroxide gel (alum), aluminum phosphate, or algannmulin, but may also be a salt of calcium, iron or zinc, or may be an insoluble suspension of acylated tyrosine, or acylated sugars, cationically or anionically derivatized polysaccharides, or polyphosphazenes.

Other adjuvants are well known in the art and include, N-acetyl-muramyl-L-threonyl-D-isoglutamine (thr-MDP), N-acetyl-normuramyl-L-alanyl-D-isoglutamine (CGP 11637, referred to as nor-MDP), N-acetylmuramyl-L-alanyl-D-isoglutaminyl-L-alanine-2-(1'-2'-dipalmitoyl-sn -glycero-3-hydroxyphosphoryloxy)-ethylamine (CGP 19835A, referred to as MTP-PE) and RIBI, which contains three components extracted from bacteria, monophosphoryl lipid A, trealose dimycolate and cell wall skeleton (MPL+TDM+CWS) in 2% squalene/Tween 80 emulsion.

Additional adjuvants can include, for example, a combination of monophosphoryl lipid A, preferably 3-de-O-acylated monophosphoryl. lipid A (3D-MPL) together with an aluminum salt. An enhanced adjuvant system involves the combination of a monophosphoryl lipid A and a saponin derivative, particularly the combination of QS21 and 3D-MPL as disclosed in PCT publication number WO 94/00153, or a less reactogenic composition where the QS21 is quenched with cholesterol as disclosed in PCT publication number WO 96/33739. A particularly potent adjuvant formulation involving QS21 3D-MPL & tocopherol in an oil in water emulsion is described in PCT publication number WO 95/17210.

The compositions and methods described herein can be employed to induce an immune response to treat and/or prevent such diseases and disorders as coccidiosis, Marek's disease, infectious bursal disease, Newcastle disease, fowl pox infection, *Clostridium spp.* infection (e.g., necrotic enteritis, gangrenous dermatitis, cholangiohepatitis, cellulites, ulcerative enteritis, botulism, Tyzzer's disease), avian influenza, infectious bronchitis, chick anemia virus infection, avian laryngotracheitis, avian metapneumovirus, avian reovirus infection, avian adenovirus infections, rotavirus infection, astrovirus infection, inclusion body hepatitis, egg drop syndrome, adenovirus infection, *Escherichia coli* infection, *Mycoplasma* spp. infection, *Salmonella* spp. infection, *Campylobacter* spp. infection, Listeria spp., *Haemophilus* spp. infection, *Pasteurella* spp.; *Bordetella* spp., *Staphylococcus* spp., *Streptococcus* spp., *Mycobacterium* spp., *Erysipelothrix* spp. and any combination thereof.

Thus, the composition described herein can also comprise an antigen or immunogen from Marek's disease virus, infectious bronchitis virus, *Mycoplasma* spp., avian leucosis virus, reovirus, poxvirus, adenovirus, cryptosporidium, chicken infectious anemia virus, *Pasteurella* species, avian influenza virus" Newcastle disease virus (NDV), infectious bursal disease virus (IBDV), Rous sarcoma virus, *Escherichia coli, Eimeria* species such as *Eimeria tenella* (causing coccidiosis), *Haemophilus* species, Mycoplasma, *Listeria* species, *Salmonella* species, *Campylobacter* species, *Clostridium* species (e.g., *C*. *perfringens, C. septicum, C. sordellii, C. difficile, C. novyi, C. botulinum, C. colinum, C. chauvoei, C. fallax, C*. *sporogenes,* and *C*. *piliforme)* and any combination thereof.

The methods and compositions described herein are intended to immunize avians against pathogens, which can be pathogens that cause disease in avians and/or pathogens that are carried by avians (contaminated avians) and are passed on to humans and other animals who handle or eat such contaminated avians. Thus, the present description discloses compositions comprising, consisting essentially of and/or consisting of a nonreplicating agent that induces an immune response against an avian pathogen or against a pathogen that causes disease in other animals by contact with or ingestion of eggs or meat or other body parts of a contaminated avian. Such pathogens can include but are not limited to Salmonella spp., *Campylobacter spp., Listeria, Escherichia coli, Erysipelothrix* spp., *Mycobacterium* spp., *Clostridium* spp., etc.

The uses of the immunogenic composition of the invention as described herein can further comprise the step of administering a booster dose of the composition of this invention to the avian subject post hatch.

In further embodiments of this invention, an effective immunizing dose of two or more compositions that induce an immune response against the avian pathogen are administered *in ovo* to the embryo, wherein the two or more compositions are administered simultaneously or sequentially in any order. Thus, the compositions of this invention can be employed using apparatus and technology that comprises administering multiple compositions at a single site, multiple compositions at multiple sites and/or a single composition at multiple sites. Such methods can employ a single entry site into the egg or multiple entry sites into the egg. Examples of such apparatus and technology are described in U.S. Patent No. 4,903,635, U.S. Patent No. 5,136,979, U.S. Patent No. RE 35,973, U.S. Patent No. 5,339,766, U.S. Patent No. 6.032,612, U.S. Patent No. 6,286,455, U.S. Patent No. 5,158,038, U.S. Patent No. 6,601,534 and U.S. Patent No. 6,981,470.

Also included herein are uses wherein an effective immunizing dose of two or more compositions that induce an immune response against the avian pathogen are administered *in ovo* and at least one composition is administered to the embryo, wherein the two or more compositions are administered simultaneously or sequentially in any order. In some of these uses, at least one composition can be administered to the amnion, which includes the amniotic fluid, embryo body and yolk sac and/or the at least one composition can be administered directly into the amniotic fluid, the embryo body and/or the yolk sac, individually or in any combination.

Some of the uses of the composition of the invention can further comprise administering *in ovo* an immune stimulant at any time during incubation, wherein the immune stimulant and the composition are administered simultaneously or sequentially in any order. The use of the composition of this invention can also further comprise administering *in ovo* a nutrient formulation, an enteric modulator, or a combination thereof at any time during incubation, wherein the nutrient formulation, the enteric modulator or a combination thereof and the composition are administered simultaneously or sequentially in any order.

There are several aspects of avian embryonic development that make the embryo an attractive target for immunization. First, since the greatest period of embryonic development occurs in the egg outside the maternal reproductive tract, the embryo can be easily accessed for the introduction of compositions such as the immunogenic compositions of this invention.

Second, the fact that the egg is a multi-compartmentalized unit can be exploited to deliver biological materials to specific embryonic sites. For example, the yolk sac in the early embryo functions to manufacture blood. Immediately prior to hatching, the yolk sac serves a primarily nutritional function and in part is taken into the intestinal tract and thereby transported to the cecal pouches during and after hatch. Therefore, yolk sac administration of materials can lead to both embryonic cecal or vascular system delivery. In addition, administration of a composition of this invention can be efficiently carried out by injection onto the chorio-allantoic membrane or onto the air cell membrane. Finally, access to the embryonic musculature compartment can be achieved by direct embryonic injection at transfer in the last quarter of incubation, and in chickens this is generally carried out from day 17 through day 19 of incubation.

The immunogenic composition may be introduced into any region of the egg, including the air cell, the albumen, the chorio-allantoic membrane, the yolk sac, the yolk, the allantois, the amnion, or directly into the embryonic bird. In particular, the composition is introduced into muscle tissue of the embryonic bird or the composition is introduced into skeletal muscle tissue. Introduction of a nucleic acid molecule encoding a protein which remains within the muscle cell can be used to administer a immunogenic protein directly and specifically to muscle cells. Alternatively, a nucleic acid molecule can be introduced which encodes a protein which will be secreted from the muscle cell and this method can be used to deliver a protein to the entire body of the bird through contact between the muscle tissue and plasma. Exemplary skeletal muscle tissue introduction sites are breast muscle and pipping muscle tissue, which are located near the eggshell and thus are relatively easily reached by injection apparatus without damage to other embryonic structures.

Any suitable means may be used for introducing the composition of this invention *in ovo,* including *in ovo* injection, high pressure spray through the egg shell, and ballistic bombardment of the egg with microparticles carrying the composition. In some embodiments, the composition is administered by depositing an aqueous, pharmaceutically acceptable solution in the muscle, which solution contains the composition to be deposited.

Where *in ovo* injection is used, the mechanism of injection is not critical, but it is preferred that the method not unduly damage the tissues and organs of the embryo or the extraembryonic membranes surrounding it so that the treatment will not decrease hatch rate. Preferred injection sites are intramuscular and subcutaneous. Preferred muscle tissue injection sites are skeletal muscle, and more particularly breast muscle and pipping muscle tissue, which are located near the eggshell and thus are relatively easily reached by injection apparatus without damage to other embryonic structures and without compromising the protection afforded by the eggshell. A syringe fitted with a needle of about 18 to 26 gauge is suitable for the purpose. Depending on the precise stage of development and position of the embryo, a ¾ to 4 inch needle will terminate either in the fluid above the chick or in the chick itself. A pilot hole may be punched or drilled through the shell prior to insertion of the needle to prevent damaging or dulling of the needle. If desired, the egg can be sealed with a substantially bacteria-impermeable sealing material such as wax or the like to prevent subsequent entry of undesirable bacteria.

The composition is administered to the embryo body in a needle having a length from about ¾ inch to about 4 inches (e.g., 1 inch, 1.25 inch, 1.5 inch, 1.75 inch, 2.0 inch, 2.25 inch, 2.5 inch, 2.75 inch, 3.0 inch, 3.25 inch, 3.5 inch, 3.75 inch, or 4.0 inch). Furthermore, a needle used in this invention can have a gauge ranging from 15g to 28g (e.g., 15g, 16g, 17g, 18g, 19g, 20g, 21g, 22g, 23g, 24g, 25g, 26g, 27g or 28g). The needle can have a blunt end and in some cases, the needle can have a beveled end with a bevel angle of about 10° to about 45° (e.g., 11°, 12°, 13°, 14°, 15°, 16°, 17°, 18°, 19°, 20°, 21°, 22°, 23°, 24°, 25°, 26°, 27°, 28°, 29°, 30°, 31°, 32°, 33°, 34°, 35°, 36°, 37°, 38°, 39°, 40°, 41°, 42°, 43°, 44°, or 45°⁾.

In particular, when administering a composition of this invention *in ovo,* the needle can pass through the shell at the large end of an egg at an angle offset by about 1° to about 20° (e.g., 1°, 2°, 3°, 4°, 5°, 6°, 7°, 8°, 9°, 10°, 11°, 12°, 13°, 14°, 15°, 16°, 17°, 18°, 19°, or 20°) from the long axis of the egg.

The composition of this invention may be administered *in ovo* with an automated injection device.

It is envisioned that a high speed automated injection system for avian embryos will be particularly suitable for practicing the present invention. Numerous such devices are available, exemplary being the EMBREX INOVOJECT^{™} system (described in U.S. Pat. No. 4,681,063 to Hebrank), and U.S. Pat. Nos. 4,040,388, 4,469,047, and 4,593,646 to Miller. The disclosure of these references and all references cited herein are to be incorporated herein by reference. All such devices, as adapted for practicing the present invention, comprise an injector containing the DNA as described herein, with the injector positioned to inject an egg carried by the apparatus with the DNA. In addition, a sealing apparatus operatively associated with the injection apparatus may be provided for sealing the hole in the egg after injection thereof.

The currently preferred apparatus for practicing the present invention is disclosed in U.S. Pat. No. 4,681,063 to Hebrank and U.S. Pat. No. 4,903,625 to Hebrank. This device comprises an injection apparatus for delivering fluid substances into a plurality of eggs and suction apparatus which simultaneously engages and lifts a plurality of individual eggs from their upwardly facing portions and cooperates with the injector for injecting the eggs while the eggs are engaged by the suction apparatus. The features of this apparatus may be combined with the features of the apparatus described above for practicing the present invention. Those skilled in the art will appreciate that this device can be adapted for injection into any portion of the egg by adjusting the penetration depth of the injector, as discussed in greater detail below.

An injection method and apparatus that can be employed in the present invention are described in US Patent No. 6,032,612 (multisite *in ovo* injection apparatus), US Patent No. 6,244,214 (apparatus for *in ovo* injection and detection of information regarding the interior of the egg), US Patent Nos. 6,176199, 6,510,811 and 6,834,615 (methods of localizing allantoic fluid in an egg), US Patent No. 7,089,879 (methods for manipulating air cells within avian eggs) and US Patent No. 7,165,507 (methods and apparatus for accurately positioning a device within the subgerminal cavity of an egg).

Thus, the method and apparatus is essentially as described in one or more of the patents listed above, and involves positioning an elongate injector or injection needle at the large end of the egg at an angle (A) offset from the long axis of said egg, the angle selected so that the needle is directed toward the shoulder or breast of said embryo. The needle is then inserted through the shell of the egg, along an essentially linear path of travel, to a depth sufficient to pass into the shoulder or breast of the embryo. The substance to be deposited in the egg, which may be either a liquid or a syringable (e.g., injectable) solid (but is generally an aqueous solution containing the composition of this invention as described herein), is then injected through the needle. In some cases, the needle is withdrawn along the essentially linear path of travel, and the step of injecting the substance is carried out concurrently with the step of withdrawing the needle so that the substance is administered along the path of travel within the egg. The angle of offset (A) is sufficient to enhance the probability of injecting into shoulder or breast muscle. Typically, the angle is 1 to 20 degrees, and preferably the angle is from 2 to 3 degrees. As one example, the needle may be inserted to a depth sufficient beneath the egg shell to pass into or pass into and through the shoulder or breast of the embryo. The apparatus may be modified to include means operably associated with the apparatus for positioning the egg at an angle with respect to the needle to achieve said angle (A), such as by mounting and positioning the needle(s) at an angle with respect to the suction apparatus.

In a particular example, the methods can be practiced with the apparatus described in U.S. Patent No. 6,244,214 to Hebrank, wherein an apparatus (e.g., a "smart probe") for identifying the specific structure and or compartment within an egg that is in contact with a needle that has penetrated the shell of the egg, and methods for employing the apparatus for delivering compositions into specific structures and/or compartments within an egg are described.

Thus, a method of introducing a substance into the muscle of a chicken *in ovo,* comprises:
a) obtaining a chicken egg, wherein said egg contains a chicken embryo in its last quarter of incubation prior to hatch; b) positioning an elongate injection needle at the large end of the egg at an angle offset about 1 to 5 degrees from the long axis of said egg, said angle selected so that the needle is directed toward the shoulder or breast of said embryo; c) inserting said needle through the shell of said egg along an essentially linear path of travel to a depth of about 7/8 inch to 1.5 inch into the shoulder or breast of said embryo; and d) injecting said substance into the egg through said needle.

Yet other methods are provided herein for introducing a substance into the muscle of a chicken *in ovo,* comprising: a) obtaining a chicken egg, wherein said egg contains a 17-19 day chicken embryo; b) positioning an elongate injection needle at the large end of the egg at an angle offset about 1 to 5 degrees from the long axis of said egg, said angle selected so that the needle is directed toward the shoulder or breast of said embryo; c) inserting said needle through the shell of said egg along an essentially linear path of travel to a depth of about 7/8 inch to 1.5 inch into the shoulder or breast of said embryo; and d) injecting said substance into the egg through said needle. In such methods, the needle can be inserted to a depth sufficient to pass into and through the shoulder or breast of said embryo.

Also provided herein is an apparatus for simultaneously injecting muscle tissue of chicken embryos in a plurality of eggs during days 17 to 19 of incubation, said device comprising: engaging means for engaging said plurality of eggs; injection means cooperating with said engaging means for inserting an elongate needle through the shells of said eggs along an essentially linear path of travel to a depth of about 7/8 inch to 1.5 inch into the shoulder or breast of said embryo; and positioning means for positioning said elongate injection needle at the large end of said egg at an angle of about 1 to 5 degrees of offset from the long axis of said egg so that said needle is directed toward the shoulder or breast of said embryo. In such an apparatus, the engaging means can comprise suction means for simultaneously lifting a plurality of individual eggs.

Compositions are provided by this invention to induce an immune response to *Clostridium* species in an avian subject. As one example, the acute enterotoxemia called necrotic enteritis in birds is caused by *Clostridium perfringens* (types A and C have been associated with the avian disease). Necrotic enteritis can occur when Clostridia-contaminated feed and litter are ingested by birds, and the organism grows in the intestine and then forms spores. This sporulation process causes the release of the alpha and beta toxins responsible for intestinal necrosis (particularly in the jejunum and ileum). Clinical signs include depression, decreased appetite and diarrhea. Acute mortality can occur. Predisposing factors for *C*. *perfringens* infection and necrotic enteritis include diet and damage to the intestinal mucosa. In the commercial poultry context, the majority of cases of necrotic enteritis occur in broiler chickens from two to five weeks of age. Thus, particular embodiments of the present invention are directed to immunogenic compositions that protect birds against *Clostridium perfringens* and necrotic enteritis, for example, by reducing infection rates and/or by reducing the severity of the infection and/or disease.

Unless otherwise defined, all technical and scientific terms used herein have the same meaning as commonly understood by one of ordinary skill in the art to which this invention belongs.

As used in the description of the invention and the appended claims, the singular forms "a," "an" and "the" are intended to include the plural forms as well, unless the context clearly indicates otherwise.

As used herein, "and/or" refers to and encompasses any and all possible combinations of one or more of the associated listed items, as well as the lack of combinations when interpreted in the alternative ("or").

Further, the term "about," as used herein when referring to a measurable value such as an amount of a compound or agent of this invention, dose, time, temperature, and the like, is meant to encompass variations of ± 20%, ± 10%, ± 5%, ± 1%, ± 0.5%, or even ± 0.1% of the specified amount.

The terms "avian" and "avian subjects" or "bird" and "bird subjects" as used herein, are intended to include males and females of any avian or bird species, and in particular are intended to encompass poultry which are commercially raised for eggs, meat or as pets. Accordingly, the terms "avian" and "avian subject" or "bird" and "bird subject" encompass chickens, turkeys, ducks, geese, quail, pheasant, parakeets, parrots, cockatoos, cockatiels, ostriches, emus and the like. In particular embodiments, the subject is a chicken or a turkey. Commercial poultry includes broilers and layers, which are raised for meat and egg production, respectively.

In the present invention, the subject is one that is at risk for or is susceptible to infection or disease caused by *Clostridium* species (e.g., necrotic enteritis caused by infection with *Clostridium perfringens*). Risk factors for necrotic enteritis are known in the art and include, but are not limited to, dietary factors (*e*.*g*., a diet high in wheat, barley, rye or fishmeal), poor litter conditions, and/or exposure to *Eimeria (e.g.,* natural exposure or live *Eimeria* vaccines). Thus, in particular, the inventive compositions can advantageously be employed to reduce the severity and/or incidence of necrotic enteritis among birds that have been vaccinated against coccidiosis or in flocks that are experiencing an outbreak of coccidiosis.

Other diseases and disorders caused by infection of avians with *Clostridium* include but are not limited to necrotic enteritis, gangrenous dermatitis, cholangiohepatitis, cellulites, ulcerative enteritis, botulism and Tyzzer's disease. Thus, the present invention provides immunogenic compositions for use to protect avians against infection by, for example, *Clostridium perfringens, C. septicum, C. sordellii, C. difficile, C. novyi, C. botulinum, C. colinum, C. chauvoei, C. fallax, C. sporogenes* and/or *C*. *piliforme.*

The avian subject can be a live, embryonic bird *in ovo* or may be a hatched bird, including newly-hatched (*i.e*., about the first one, two or three days after hatch), adolescent, and adult birds.

In particular, the bird is about six-, five-, four-, three-, two- or one-week of age or less. Also the avian subject can be a naïve subject, *i*.*e*., has not previously been exposed to the antigen against which immunity is desired.

The term "administering *in ovo*" or "*in ovo* administration," as used herein, unless otherwise indicated, means administering an immunogenic composition (*e*.*g*., a vaccine) to a bird egg containing a live, developing embryo by any means of penetrating the shell of the egg and introducing the immunogenic composition. Such means of administration include, but are not limited to, *in ovo* injection of the immunogenic composition.

The present invention provides use of an immunogenic composition administered to a subject to induce an immune response against *Clostridium* species, optionally a protective immune response, in the subject. The immunogenic composition can be administered to any suitable compartment of the egg (*e*.*g*., allantois, yolk sac, amnion, air cell and/or into the avian embryo itself), as would be apparent to one skilled in the art. Methods of administration into the embryo include without limitation parenteral administration, such as for example, subcutaneous, intramuscular, intra-abdominal, intravenous, and/or intra-articular administration. In particular, the immunogenic composition is administered to the amnion (*e*.*g*., by injection axially through the large end of the egg).

The immunogenic composition can be administered to the egg by injection. The mechanism of egg injection is not critical, but generally should be selected so that the method does not damage the tissues and organs of the embryo or the extraembryonic membranes surrounding it to such an extent that the treatment unduly decreases hatch rate. A syringe fitted with a needle of about 18 to 23 gauge is generally suitable for the purpose. Examples of needles suitable for use in this invention include needles having the following gauges: 18, 19, 20, 21, 22, or 23 gauge. A needle for use in this invention can be at least ½ inch, 5/6 inch, % inch, 7/8 inch, 1 inch, 1 and ¼ inch, 1 and 3/8 inch, 1 and ½ inch, 1 and 5/8 inch, 1 and ¾ inch, 1 and 7/8 inch or 2.0 inches in length. Examples of a bevel range of a needle is from about 5 degrees to about 45 degrees (e.g., 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 45, 56, 37, 38, 39, 40, 41, 42, 43, 44 or 45 degrees) In particular, the bevel range of a needle of this invention is from about 12 degrees to 20 degrees. A pilot hole can be punched or drilled through the shell prior to insertion of the needle to prevent damaging or dulling of the needle. If desired, the egg can be sealed with a substantially bacteria-impermeable sealing material such as wax or the like to prevent subsequent entry of undesirable bacteria.

A high-speed automated egg injection system for avian embryos is particularly suitable for practicing the present invention. Numerous such devices are available, exemplary being those disclosed in U.S. Patent Nos. 4,681,063 and 4,903,635 to Hebrank and U.S. Patents Nos. 4,040,388, 4,469,047, and 4,593,646 to Miller. Such devices, as adapted for practicing the present invention, generally comprise an injector containing the immunogenic composition, with the injector positioned to inject an egg carried by the apparatus with the immunogenic composition. In addition, if desired, a sealing apparatus operatively associated with the injection apparatus may be provided for sealing the hole in the egg after injection thereof.

In one embodiment, the apparatus for practicing the present invention can be as disclosed in U.S. Pat. No. 4,681,063 to Hebrank and U.S. Pat. No. 4,903,625 to Hebrank. This device comprises an injection apparatus for delivering fluid substances into a plurality of eggs and suction apparatus which simultaneously engages and lifts a plurality of individual eggs from their upwardly facing portions and cooperates with the injector for injecting the eggs while the eggs are engaged by the suction apparatus. Those skilled in the art will appreciate that this device can be adapted for injection into any portion of the egg by adjusting the penetration depth of the injector, as is known in the art. The present invention can also be practiced with the apparatus and methods described in U.S. Patent No. 6,244,214 to Hebrank, wherein an apparatus for identifying the specific structure and or compartment within an egg that is in contact with a needle that has penetrated the shell of the egg and methods for employing the apparatus for delivering compositions into specific structures and/or compartments within an egg are described.

The appropriate volume of the immunogenic composition to be administered will depend on the size of the egg being treated, with ostrich eggs obviously being capable of taking more volume than chicken eggs. In particular, the immunogenic composition is administered in a volume from about 10 to about 500, 1000 or 2000 µl or more, including any number between 10 and 2000, even if not explicitly recited herein, with exemplary volumes including 10, 20, 30, 40, 50, 60, 70, 80, 90, 100, 125, 150, 175, 200, 225, 250, 275, 300, 325, 350, 375, 400, 425, 450, 475, 500, 525, 550, 575, 600, 626, 650, 675, 700, 725, 752, 775, 800, 850, 900, 1000, 1100, 1200, 1300, 1400, 1500, 1600, 1700, 1800, 1900 or 2000 µl. Other suitable volumes for delivering the immunogenic composition can be readily determined by those skilled in the art.

In particular, the eggs (*i.e.,* embryonic birds) administered the immunogenic composition are in the last half or the last quarter of *in ovo* incubation (*i*.*e*., of embryonic development). For example, for chicken eggs the last half of incubation is from about the twelfth to twentieth day of incubation (e.g., E12, E13, E14, E15, E16, E17, E17.5, E18, E18.5, E19, E19.5 and/or E20), and the last quarter of *in ovo* incubation is from about the fifteenth to twentieth day of incubation (e.g., E15, E15.5, E16, E16.5, E17, E17.5, E18, E18.5, E19 E19.5 and/or E20). In particular, the egg is administered the immunogenic composition on about the eighteenth (E18 or E18.5) or nineteenth (E19 or E19.5) day of *in ovo* incubation. Turkey eggs are administered the immunogenic composition on about the fourteenth to twenty-seventh day of incubation (E14, E15, E16, E17, E18, E19, E20, E21, E21.5, E22, E22.5, E23, E23.5, E24, E24.5, E25, E25.5, E26 and/or E27), on about the twenty-first to twenty-seventh day of incubation (e.g., E21, E21.5, E22, E22.5, E23, E23.5, E24, E24.5, E25, E25.2, E26, E26.5 and/or E27), or on about the twenty-fifth (E25 or E25.5) day of incubation. Those skilled in the art will appreciate that the present invention can be carried out at any predetermined time *in ovo,* as long as the administration results in a desired immune response to the immunogenic composition without undue levels of morbidity and/or mortality among the treated subjects.

An immunogenic composition also can be administered to a hatched bird, including newly hatched (*i.e.,* about the first one, two and/or three days after hatch), adolescent, and/or adult birds. Administration is within about the first six, five, four, three and/or two weeks after hatch and/or even within about the first week after hatch, preferably administration is within the first three weeks after hatch. The immunogenic composition of the present invention is administered *in ovo* (*e*.*g*., in the last quarter of *in ovo* incubation) and a booster is administered post-hatch (*e*.*g*., within about the first one, two or three days or one, two or three weeks post-hatch).

The present invention can be distinguished from maternal vaccination approaches in which older female birds (*e*.*g*., hens at about 10-15 weeks of age) are vaccinated for the purpose of providing passive immunity to their offspring. Such birds are probably not naive, *i.e.,* they have already been exposed to *Clostridium* (e.g., *Clostridium perfringens)* and are not being immunized for the purpose of protecting the vaccinated bird but instead to protect the offspring by passive transfer of antibodies.

Immunogenic compositions as described herein can be administered to hatched birds by any suitable means. Exemplary means are oral administration (e.g., in the feed or drinking water), intramuscular injection, subcutaneous injection, intravenous injection, intra-abdominal injection, eye drop and/or nasal spray. Further, birds can be administered immunogenic compositions in a spray cabinet, *i.e.,* a cabinet in which the birds are placed and exposed to a mist containing vaccine, or by course spray.

The invention can be practiced to protect a bird from necrotic enteritis. By "protect," "protecting," and "protection" and like terms it is meant any level of protection from necrotic enteritis which is of some benefit in a population of subjects, such that there is a reduction in the incidence and/or the severity of the disease among treated birds whether in the form of decreased mortality, decreased lesions, improved body weight, improved feed conversion ratios and/or the reduction of any other detrimental effect of the disease, regardless of whether the protection is partial or complete. Those skilled in the art will understand that protection can be assessed from a plurality of treated birds as compared with untreated birds, even if individual treated birds are not protected. The invention can be practiced to reduce the incidence of necrotic enteritis among a plurality of birds that are administered the immunogenic compositions of the invention. Morbidity and/or mortality can be reduced among a plurality of birds that are treated according to the present invention.

By "prime," "primed" or "priming" (and grammatical variations thereof) as used herein, it is meant to initiate an active immune response that is less than the protective until a second dose (booster) has given at a later time post hatch.

By "reduce," "reduced," "reducing," and "reduction" (and grammatical variations thereof), as used herein, it is meant a decrease in the indicated infection- or disease-related parameter (*e*.*g*., infection, morbidity, mortality, incidence of necrotic enteritis, lesions and the like) that is of some value or benefit to the user (*e*.*g*., commercial value), for example, a decrease of at least about 20%, 25%, 35%, 50%, 75%, 80%, 85%, 90%, 95%, 97% or more as compared with untreated birds.

The immunogenic compositions used in the invention protect birds from infection by *Clostridium* species, which results in any level of protection that is of some benefit in a population of subjects, such that there is a reduction in the incidence and/or the severity of *Clostridium* infection among treated birds.

The invention is practiced to induce an immune response to *Clostridium.* As used herein, the term "induce (or grammatical variations thereof) an immune response against *Clostridium"* is intended to encompass agents that induce an immune response against the organism itself and/or the toxins produced by the organism, by means of passive transfer or active immune response. Optionally, the immune response that is induced is a protective immune response, for example, in vaccination methods. Protection is not required if there is some other purpose for inducing the immune response, for example, for research purposes or to produce antibody for passive immunizations or as a reagent (*e*.*g*., to detect, isolate and/or identify *Clostridium* species).

As used herein, unless indicated otherwise, "C. *perfringens"* is intended to include *C*. *perfringens* type A and/or *C*. *perfringens* type C and/or any other *C. perfringens* type that is implicated in the etiology of necrotic enteritis in birds. In particular embodiments, the invention provides methods of protecting birds from infection by *C*. *perfringens* type A and/or *C*. *perfringens* type C. The invention also provides methods of inducing an immune response against *C*. *perfringens* type A and/or *C*. *perfringens* type *C*. Different types of *C*. *perfringens* and strains thereof are well-known in the art. *See*, *e*.*g*., AMERICAN ASSOCIATION OF AVIAN PATHOLOGISTS, A LABORATORY MANUAL FOR THE ISOLATION AND IDENTIFICATION OF PATHOGENS (3d. ed. 1989).

The term "effective immunizing dose," as used herein, unless otherwise indicated, means a dose of the immunogenic composition sufficient to induce a protective immune response in the treated birds that is greater than the inherent immunity of non-immunized birds. In the case of birds treated *in ovo,* an "effective immunizing dose" indicates a dose sufficient to induce a protective immune response in the hatched birds that have been treated *in ovo* that is greater than the inherent immunity of birds that were not immunized *in ovo.* An effective immunizing dose in any particular context can be routinely determined using methods known in the art.

An "effective immunizing dose" can comprise one or more (*e*.*g*., two or three) doses of the immunogenic composition so as to achieve the desired level of protection. The individual doses can be administered *in ovo* and/or post-hatch.

As discussed above, it will be apparent to those skilled in the art that when treating a plurality of birds (such as in commercial poultry production), the effectiveness of the dose and/or the immunogenic composition can be assessed by evaluating the effects of vaccination on the flock as a whole. In other words, an effective immunizing dose or an effective vaccine for the flock as a whole may nonetheless not induce an immune response and/or provide sufficient protection against disease in individual birds.

The terms "vaccination" or "immunization" are well-understood in the art, and are used interchangeably herein. For example, the terms vaccination or immunization can be understood to be a process that increases a subject's immune reaction to antigen (by providing an active immune response), and therefore its ability to resist, overcome and/or recover from infection (*i*.*e*., a protective immune response).

The terms "protective immunity" or "protective immune response," as used herein, are intended to mean that the host animal mounts an active immune response to the immunogenic composition and/or that the immunogenic composition provides passive immunity, such that upon subsequent exposure or a challenge, the animal is able to resist or overcome infection and/or disease. Thus, a protective immune response will decrease the incidence of morbidity and/or mortality from subsequent exposure to the pathogen among treated birds.

An "active immune response" or "active immunity" is characterized by "participation of host tissues and cells after an encounter with the immunogen. It involves differentiation and proliferation of immunocompetent cells in lymphoreticular tissues, which lead to synthesis of antibody or the development of cell-mediated reactivity, or both." Herbert B. Herscowitz, Immunophysiology: Cell Function and Cellular Interactions in Antibody Formation, in IMMUNOLOGY: BASIC PROCESSES 117 (Joseph A. Bellanti ed., 1985). Alternatively stated, an active immune response is mounted by the host after exposure to immunogens by infection or by vaccination. Active immunity can be contrasted with passive immunity, which is acquired through the "transfer of preformed substances (antibody, transfer factor, thymic graft, interleukin-2) from an actively immunized host to a non-immune host." *Id.*

Models of necrotic enteritis (NE) for assessing efficacy of vaccines and vaccination strategies are known in the art. For example, Hofacre et al. (2003, J. Appl. Poult. Res. 12:60-64) described a model in which chickens were fed a corn soy diet with 26% fishmeal from day 0 to day 14 post-hatch. Fishmeal was removed from the diet at day 14. Birds were challenged with coccidia by oral gavage at day 14, then daily from days 17-19 with *C*. *perfringens* by oral gavage. Feed conversion ratio, body weight and scoring of gut lesions were used to assess the presence and severity of necrotic enteritis in challenged birds. NE lesions were assessed on day 22 or day 28, using a scale of 0=none, 1 =mild, 2=moderate and 3=marked/severe. Other models can be used to assess vaccine efficacy and vaccine regimens as known in the art. In certain embodiments of the present invention, the administration of a composition of this invention (e.g., an effective amount of a composition of this invention) can result in about a 5, 10, 15, 20, 25, 30, 35, 40, 45, 50, 60, 70, 75, 80, 90, or 100% reduction in gut lesions and/or change in body weight in animals of this model or other known or art-accepted model, as compared to non-immunized or control animals.

The compositions of this invention can be used to induce an antibody response in avians that is at least greater than or equal to about 0.5 antitoxin units/mL (e.g., at least about 0.5, 1.0, 1.5, 2.0, 2.5, 3.0, 3.5, 4.0, 4.5, 5.0, 5.5, 6.0, 6.5, 7.0, 7.5. 8.0, 8.5, 9.0, 9.5 or 10 A.U. of anti-toxin antibody per mL of antisera of the avian).

Where the compositions of this invention are employed, the percentage of eggs of a population of eggs into which a composition of this invention is delivered into the embryo body can be from about 70% to about 100% (e.g., 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or 100%) of the total number of eggs in the population of eggs to which the composition is administered.

The present invention encompasses immunogenic compositions that induce an active and/or passive immune response against *C*. *perfringens* (including types A and/or C), and which optionally can be used to protect a bird against *C*. *perfringens* infection and/or to protect against necrotic enteritis as described in more detail above.

The immunogenic compositions of the invention comprise, consist essentially of, and/or consist of an agent(s) that induces an immune response against *Closfridium.* The immune agent of *Clostridium* can be a replicating antigen and/or a nonreplicating antigen. The replicating and nonreplicating antigens of this invention can be delivered *in ovo* to the amnion, to the embryo and/or to both the amnion and embryo.

Furthermore, the immunogenic compositions of the invention comprise, consist essentially of, or consist of an effective immunizing dose of a *Clostridium* immunizing agent in a pharmaceutically acceptable carrier. In representative embodiments, the immunogenic composition can be formulated with *Clostridium* toxoids and/or bacterins. According to this embodiment, the immunogenic composition optionally further comprises an adjuvant (see below). Toxoids are inactivated toxins, and can be derived from *Clostridium* toxins, including Toxoids are inactivated toxins, and can be derived from *Clostridium* toxins, including those derived from *C*. *perfringens,* including alpha toxin, beta toxin, beta 2 toxin, enterotoxin, epsilon toxin, iota toxin, kappa toxin, lambda toxin, and/or theta toxin; those derived from *C*. *sordellii,* including hemorrhagic toxin and/or lethal toxin; those derived from *C*. *difficile,* including A toxin (enterotoxin) and B toxin (cytopathic toxin); those derived from *C*. *septicum,* including alpha toxin; those derived from *C*. *novyi,* including alpha toxin and/or beta toxin; and/or those derived from *C*. *botulinum,* including toxin type C. Methods of producing toxoids are known in the art and include, for example, formaldehyde or heat treatment of toxin (see, *e*.*g*., Walker, (1992) Vaccine 10:977-990). Bacterins are bacterial cellular components and can be derived from a *Clostridium* species, such as, for example, from *C*. *perfringens* types A and/or *C*. *perfringens* type C. *C*. *perfringens* toxoid vaccines are known in the art (*see*, *e.g.,* U.S. Patent No. 4,292,306 to Zemlyakova).

In other embodiments, the immunogenic composition comprises, consists essentially of, or consists of a killed (i.e., nonreplicating) *Clostridium* bacterium (*i*.*e*., a bacterin), optionally in a water-in-oil-in-water emulsion (see, *e.g.,* U.S. Patent No. 5,817,320 to Stone describing *in ovo* immunization of avian embryos with oil emulsion vaccines), and/or a pharmaceutically acceptable carrier. In other embodiments, the immunogenic composition comprises, consists essentially of, or consists of killed *Clostridium* and an adjuvant (*e*.*g*., an aluminum derived adjuvant such as aluminum hydroxide, a saponin such as Quil-A including QuilA QS21, or an oil such as complete or incomplete Freund's), optionally in a water-in-oil-in-water emulsion and/or a pharmaceutically acceptable carrier.

As a further alternative described herein, the immunogenic composition comprises, consists essentially of, or consists of a replicating immune agent of *Clostridium,* e.g., live *C. perfringens,* which is generally a live attenuated (*i.e.,* with reduced virulence) *C. perfringens.* (See, for example PCT Publication No. PCT WO 2005/053737 for teachings on the production of live attenuated bacteria for vaccine use.) Methods of producing attenuated bacteria are known in the art and include without limitation: irradiation, chemical treatment, serial passage in culture, and the like. For example, live *Clostridium* bacteria (*e*.*g*., *C. perfringens)* are administered in the presence of an agent that protects the subject from the pathological effects of the organism, for example, by co-administration of a neutralizing factor as described in U.S. Patent No. 6,440,408 to Thoma et al., or interferon as described in U.S. Patent No. 6,506,385 to Poston et al. Optionally, the *Clostridium* and the neutralizing factor and/or interferon are administered in the same formulation.

Further examples of immunogenic compositions include immunogenic compositions comprising, consisting essentially of, or consisting of antitoxins (*i*.*e*., antibodies that provide passive immunity against *Clostridium* alpha and/or beta toxins; *see, e.g.,* U.S. Patent No. 5,719,267 to Carroll et al.), antigenic peptides that induce an immune response against *Clostridium* (including *C*. *perfringens* toxins; *see e.g.,* U.S. Patent Nos. 5,817,317 and 5,851,827 to Titball et al.; U.S. Patent No. 6,610,300 to Segers et al.; U.S. Patent No. 5,695,956 to McClane et al.), and recombinant vaccines that comprise a carrier nucleic acid (*e*.*g*., a plasmid or virus) that delivers a nucleic acid encoding an antigenic peptide(s) or protein(s) that induces an immune response against *Clostridium.*

In representative embodiments, the immunogenic composition comprises, consists essentially of, or consists of a recombinant alpha and/or beta toxin of *Clostridium,* for example, the alpha toxins having the amino acid sequence as shown in SEQ ID NO:2 [full-length sequence of 370 amino acids; GenBank Accession No. 1GYGB (Gl:21730290), the coding sequence of which is provided herein as SEQ ID NO:1], SEQ ID NO:4 (Cpa_{247-370;} amino acids 247-370 of SEQ ID NO:2; the coding sequence of which is provided herein as SEQ ID NO:3), SEQ ID NO:6 (amino acids 1-278 of SEQ ID NO:2; the coding sequence of which is provided herein as SEQ ID NO:5), SEQ ID NO:8 (Cpa_{261-300;} amino acids 261-300 of SEQ ID NO:2 the coding sequence of which is provided herein as SEQ ID NO:7) as described herein and/or for example, in U.S. Patent Nos. 5,817,317 and 5,851,827 to Titball et al., or SEQ ID NO:10 [full length sequence of 398 amino acids, the coding sequence of which is provided herein as SEQ ID NO:9]. In particular, the toxin of the present invention is an immunogenic composition comprising amino acids 1-278 (SEQ ID NO:6) of the 370 amino acid sequence (SEQ ID NO:2) of the alpha toxin of *C*. *perfringens.*

Further examples of toxins (including immunogenic fragments thereof) that can be used in the compositions of this invention include but are not limited to, *C*. *perfringens* toxins [e.g., alpha toxin, Accession number CAA35186 (Saint-Joanis et al. Mol. Gen. Genet. 219(3):453-460 (1989)0; beta toxin, Accession number CAA58246 (Steinthorsdottir et al. FEMS Microbiol. Lett. 130(2-3):273-278 (1995)); beta 2 toxin, Accession number NP_{_}150010 (Shimizu et al. Proc. Natl. Acad. Sci. U.S.A. 99(2):996-1001 (2002)); enterotoxin, Accession number BAE79112 (Miyamoto et al. J. Bacteriol. 188(4):1585-1598 (2006)); epsilon toxin, Accession number AAA23236 (Havard et al. FEMS Microbiol. Lett. 97:77-82 (1992); iota toxin, Accession number CAA51959 (Perelle et al. Infect. Immun. 61(12):5147-5156 (1993)); kappa toxin, Accession number NP_561089, Shimizu et al. Proc. Natl. Acad. Sci. U.S.A. 99(2):996-1001 (2002)); lambda toxin, Accession number CAA35187 (Saint-Joanis et al. Mol. Gen. Genet. 219(3):453-460 (1989)); and theta toxin, Accession number NP_561079 (Shimizu et al. Proc. Natl. Acad. Sci U.S.A. 99(2):996-1001 (2002))], *C. difficile* toxins [e.g., A toxin, accession number A37052 (Wren et al. FEMS Microbiol. Lett 70:1-6(1990)); and B toxin, Accession number CAA43299 (von Eichel-Streiber et al. Mol. Gen. Genet. 233(1-2): 260-268 (1992))], *C. septicum* toxins [alpha toxin, Accession number AAB32892 (Ballard et al. Infect. Immun. 63(1):340-344 (1995))] and *C. novyi* toxins [e.g., alpha toxin, Accession number AAB27213 (Ball et al. Infect. Immun. 61(7):2912-2918 (1993))].

The terms "toxin," "alpha toxin," "beta toxin," "epsilon toxin," (or a like term), etc., as used herein include the full-length toxin as well as antigenic or immunogenic peptides or antigenic or immunogenic variants (*e*.*g*., attenuated) thereof that induce an immune response (optionally, a protective immune response) against *Clostridium* in the subject. In particular, an antigenic peptide comprises at least about 6, 8, 10, 12, 15, 18, 20, 25, 30, 50, 75 or 100 or more contiguous amino acids of the full-length toxin (*see*, *e.g*., the full-length alpha toxin sequence as shown in SEQ ID NO:2 in U.S. Patent Nos. 5,817,317 and 5,851,827 and in SEQ ID NO:2 herein).

It is also understood that the immunogenic fragments used in the compositions of this invention can be combined in any order or amount. For example, fragment 1-10 can be combined with fragment 10-20 to produce a fragment of amino acids 1-20. As another example, fragment 1-20 can be combined with fragment 50-60 to produce a single fragment for use in the compositions of this invention having 31 amino acids (AA 10-20 and AA 50-60). Also fragments can be present in multiple numbers and in any combination in a fragment for use in the compositions of this invention. Thus, for example, fragment 1-150 can be combined with a second fragment 1-150 and/or combined with fragment 400-500 to produce a fragment for use in the compositions of this invention.

An antigenic or immunogenic fragment of a *Clostridium* toxin of this invention can comprise, consist essentially of and/or consist of the amino terminal domain of *C*. *perfringens* alpha toxin (amino acids 1-246 of SEQ ID NO:2), the carboxy terminal domain of *C*. *perfringens* alpha toxin (amino acids 256-370 of SEQ ID NO:2) and/or the fragment between these domains (amino acids 247-255 of SEQ ID NO:2) in any combination and with any amount of overlap in amino acid sequence that results in a fragment having immunogenic activity. This language is intended to encompass all possible toxin peptides and fragments as if explicitly set forth herein (*e*.*g*., any peptide or fragment comprising at least about 6, 8, 10, 12, 15, 18, 20, 25, 30, 50, 75 or 100 or more contiguous amino acids of the full-length alpha toxin sequence as shown in SEQ ID NO:2 in U.S. Patent Nos. 5,817,317 and 5,851,827 and in SEQ ID NO:2 and SEQ ID NO:10 herein). In particular, the antigenic peptide lacks an amino acid sequence having phospholipase C and/or sphingomyelin hydrolyzing activity (*e*.*g*., an antigenic alpha toxin peptide can lack amino acids 1-240). The location of some *C*. *perfringens* alpha toxin epitopes has been determined (*see, e.g.,* Logan et al., (1992) Infection and Immunity 59:4338-4382).

Additional examples of recombinant *Clostridium* toxins that can be employed in this invention include, but are not limited to, a *Clostridium perfringens* beta toxin or an immunogenic fragment thereof, wherein the beta toxin has the amino acid sequence as set forth in SEQ ID NO:11. The beta toxin of SEQ ID NO:11 can further comprise a mutation at amino acid 62, 182, 197 or in one of the regions between amino acid numbers 80-103, 145-147, 281-291, 295-299 or downstream of amino acid position 292 (as described in U.S. Patent No. 6, 610,300), whereby the resulting toxin or fragment thereof has immunogenic activity.

The nucleic acid and amino acid sequences of *C*. *perfringens* alpha and beta toxins are known in the art, see, *e*.*g*., GenBank Accession Nos. DQ202275; NP_560952; NC_003366; AY823400; AY277724; AF204209; X17300; X13608; L43548; L43547; L77965 and L13198. *See also*, Sheedy et al., Highly Conserved Alpha-Toxin Sequences of Avian Isolates of Clostridium perfringens, J. Clin. Microbiol. 42:1345-1347 (2004) presenting an analysis of the alpha toxin sequences of 25 chicken-derived *C*. *perfringens* strains.

The *Clostridium* toxin can be an epsilon (ε) toxin of *C*. *perfringens,* having an amino acid sequence as set forth in SEQ ID NO:12 (328 amino acids; or SEQ ID NO:13. Also, the ε toxin can comprise the amino acid sequence of SEQ ID NO:13, wherein residue 2 is a proline, as described in U.S. Patent No. 6,403, 094.

The present invention provides use of an immunogenic composition for immunizing an avian subject against infection by *Clostridium,* comprising administering to the avian subject an effective immunizing dose of a *Clostridium* bacterin-toxoid composition by *in ovo* injection during the final quarter of incubation. The invention can further comprise the step of administering a booster dose of the *Clostridium* bacterin-toxoid composition to the avian subject post hatch. The *Closfridium* species of this invention can include, but is not limited to *Clostridium perfringens.* In particular, the composition can comprise a Vision CD^{®} vaccine. In particular wherein the subject is a chicken, the bacterin-toxoid composition can be administered into the amniotic fluid via a 20g, 1.0 inch needle at day 18 of incubation or a 22g, 1.0 inch needle during day 18 of incubation.

In addition, uses are provided of immunizing an avian subject against infection by *Clostridium,* comprising administering to the avian subject an effective immunizing dose of a recombinant toxin or immunogenic fragment thereof of *Clostridium* by *in ovo* injection during the final quarter of incubation. These uses can further comprise the step of administering a booster dose of the recombinant toxin or immunogenic fragment thereof to the avian subject post hatch. In particular embodiments, the composition employed in these uses can comprise an adjuvant, which can be Quil A and incomplete Freund's adjuvant. In embodiments wherein the subject is a chicken, the bacterin-toxoid composition can be administered into the embryo body via a 23g, 1.25 inch needle during day 19 of incubation.

When the subject is a chicken, a toxin or immunogenic fragment thereof of this invention can be administered into the embryo body via a 20g, 1.5 inch needle during day 19 of incubation. Also, the dosage range of a toxin (e.g., an alpha toxin) or immunogenic fragment thereof and/or other subunit protein or glycoprotein or other type of biological molecule used as a vaccine of this invention can be from about 1 µg to about 1000 µg per dose, with an exemplary range of about 55 µg to about 60 µg per dose. For compositions of this invention comprising inactivated virus, the virus concentration per dose can be about 10³EID₅₀/TCID₅₀ to about 10¹² EID₅₀/TCID₅₀ (EID = egg infectious dose; TCID = tissue culture infectious dose). For activated virus, the virus concentration per dose would be about 10^{0.1} EID₅₀/TCID₅₀ to about 10¹² EID₅₀/TCID₅₀. In particular, the toxin of this invention comprises, consists essentially of and/or consists of the amino acid sequence of SEQ ID NO:2, 4, 6, 8 or 10, including any combination thereof.

As contemplated herein, in some embodiments of the present invention, the composition of this invention further comprises an adjuvant, which in particular embodiments, can be an adjuvant such as an aluminum derived adjuvant (*e*.*g*., aluminum hydroxide), a saponin (*e*.*g*., Quil-A including QuilA QS21), or an oil (such as Complete or Incomplete Freund's adjuvant), in any combination. Additional examples of adjuvants that can be employed in any of the methods of the inventions described herein are provided herein.

In representative embodiments, the immunogenic composition of this invention comprises, consists of, of consists essentially of a *C*. *perfringens* enterotoxin (CPE), beta-2 toxin, epsilon toxin, kappa toxin, lambda toxin, theta toxin, and/or iota toxin, optionally in addition to a *C*. *perfringens* alpha and/or beta toxin.

In other representative embodiments, the immunogenic composition comprises, consists essentially of, or consists of a toxoid or toxoid/bacterin. The bacterin can be a *C*. *perfringens* type A and/or type C bacterin. For example, an exemplary immunogenic composition comprises, consists essentially of, or consists of an alpha toxoid and a *C*. *perfringens* type A bacterin. Optionally, the immunogenic composition further comprises an adjuvant such as an aluminum derived adjuvant (*e*.*g*., aluminum hydroxide), a saponin *(e.g.,* Quil-A including QuilA QS21), or an oil (such as Complete or Incomplete Freund's adjuvant).

A representative immunogenic composition of the invention comprises, consists essentially of, or consists of an effective immunizing dose of a *C*. *perfringens* immunizing agent in a water-in-oil-in-water emulsion (*see*, *e.g.,* U.S. Patent No. 5,817,320 to Stone), optionally in a pharmaceutically acceptable carrier.

The immunogenic composition can optionally comprise two or more agents that induce an immune response against *C*. *perfringens* (*e.g*., any combination of the agents described above).

In particular, the agent that induces an immune response against *C*. *perfringens (e.g.,* a toxoid, bacterin, attenuated *C*. *perfringens,* and/or toxin and the like) is an avian-derived, optionally a chicken-derived, strain of C. *perfringens.*

As used herein, the term "consists essentially of" (and grammatical variants) means that the immunogenic composition comprises no other material immunogenic agent other than the indicated agents. The term "consists essentially of" does not exclude the presence of other components such as adjuvants, immunomodulators, and the like.

By "pharmaceutically acceptable" it is meant a material that is not biologically or otherwise undesirable, *i.e.,* the material may be administered to a subject without causing appreciable undesirable biological effects. Thus, such a pharmaceutical composition may be used, for example, to prepare compositions for immunization. Physiologically and pharmaceutically acceptable carriers may contain other compounds including but not limited to stabilizers, salts, buffers, adjuvants and/or preservatives (e.g., antibacterial, antifungal and antiviral agents) as are known in the art. The pharmaceutically acceptable carrier need not be sterile, although it generally will be for *in ovo* administration to avian embryos.

In particular embodiments, the immunogenic composition further comprises an immune stimulant. Alternatively, the immune stimulant can be administered to the subject in a separate formulation. Immune stimulants that can be used in the present invention include, but are not limited to, cytokines, growth factors, chemokines, supernatants from cell cultures of lymphocytes, monocytes, or cells from lymphoid organs, cell preparations or cell extracts (e.g., fixed *Staphylococcus aureus* or lipopolysaccharide preparations), mitogens, or adjuvants, including low molecular weight pharmaceuticals. Immune stimulants can be administered *in ovo* at any time during incubation. Optionally, the immune stimulant and the agent that induces an immune response against C. *perfringens* are administered concurrently, optionafly in the same formulation.

As used herein, the word "concurrently" means sufficiently close in time to produce a combined effect (that is, concurrently can be simultaneously, or it can be two or more events occurring within a short time period before and/or after each other).

Any suitable vaccine adjuvant can be used according to the present invention, including chemical and polypeptide immunostimulants that enhance the immune system's response to antigens. Adjuvants include but are not limited to an aluminum derived adjuvant (*e*.*g*., aluminum hydroxide), aluminum phosphate, plant and animal oils (*e.g*., incomplete or complete Freund's), saponin (*e*.*g*., Quil-A including QuilA QS21), Spur^{®} (Intervet), and the like. Representative adjuvants of this invention include but are not limited to an aluminum salt such as aluminum hydroxide gel (alum), aluminum phosphate, or algannmulin, but may also be a salt or mineral gels of calcium, magnesium, iron or zinc, or may be an insoluble suspension of acylated tyrosine, or acylated sugars, cationically or anionically derivatized polysaccharides, or polyphosphazenes., or saponins such as Quil-A, or oil emulsions such as water-in-oil and water-in-oil-in water or complete or incomplete Freund's or any combination thereof

The immunogenic composition can optionally contain one or more stabilizers. Any suitable stabilizer can be used, including carbohydrates such as sorbitol, mannitol, starch, sucrose, dextrin, or glucose; proteins such as albumin or casein; and buffers such as alkaline metal phosphate and the like.

It is often convenient to immunize a bird against multiple diseases in a single course of treatment. Thus, in particular, the immunogenic composition comprises one or more additional agents that induce an immune response against other avian pathogens (*e*.*g*., viral, bacterial or fungal), optionally immunizing agents that produce a protective immune response. For example, the immunogenic composition can further comprise an immunizing agent against coccidiosis (*i*.*e*., *Eimeria*)*,* infectious bursal disease, Marek's disease, Newcastle disease, avian influenza, fowl pox, avian reovirus, avian metapneumovirus, avian adenovirus, infectious bronchitis, *Salmonella spp., Camplyobacter spp., Pasteurella spp., Hemophilus paragallinarum* and/or *Mycoplasma spp.* Avian vaccines suitable for *in ovo* or post-hatch use are known in the art and are commercially available (*e*.*g*., Bursaplex™ vaccine for bursal disease; Newplex™ vaccine for Newcastle disease, and Inovocox™ vaccine for coccidiosis, all available from Embrex, Inc., and Marek's HVT-SB-1 vaccine for Marek's disease, available from Merial). Immunogenic compositions comprising vaccine agents against both coccidiosis *(i.e., Eimeria)* and necrotic enteritis (*i.e*., *C. perfringens*) are particularly advantageous because *Eimeria* exposure is known to increase the susceptibility of birds to necrotic enteritis by perturbing the gastrointestinal environment.

Thus, as a further aspect, the invention encompasses co-administering an immunogenic composition that comprises, consists essentially of, or consists of an effective immunizing dose of a *C*. *perfringens* immunizing agent and an effective immunizing dose of an immunizing agent that provides protection against one or more other avian diseases (as described above). The multiple immunizing agents can be provided in a single formulation or can be administered concurrently or sequentially in any order in separate formulations. As discussed above, this aspect of the invention is particularly suited to co-administration of coccidiosis and necrotic enteritis vaccines.

The avian subject may be first immunized against necrotic enteritis and then immunized against coccidiosis or vice versa. The immunizations can both occur *in ovo,* both can occur post-hatch, or one can be *in ovo* and one post-hatch. For example, the avian subject is immunized against coccidiosis *in ovo* and then is immunized against necrotic enteritis after hatch.

The invention can also be practiced to administer a *C*. *perfringens* immunizing agent *in ovo* in conjunction with *"in ovo* feeding" (see, U.S. Patent No. 6,592,878) of the avian subject. For example, according to certain embodiments, a *C*. *perfringens* immunizing agent and a nutrient formulation and/or enteric modulator are administered to an avian subject *in ovo,* optionally by delivery to the amnion. Optionally, vaccines against other infectious agents are administered *in ovo* and/or post-hatch as well (as described above). The *C*. *perfringens* immunizing agent and the nutrient formulation and/or enteric modulator can be administered concurrently, in the same or separate compositions, and/or can be administered sequentially in any order.

Further embodiments of the present invention can include a composition comprising an antigen selected from the group consisting of a *C*. *perfringens* alpha toxoid, an antigenic fragment of a *C*. *perfringens* alpha toxoid, an inactive antigenic fragment of a *C*. *perfringens* alpha toxin, and any combination thereof; wherein one or more doses of about 0.1 to about 1.0 mL (e.g., 0.1, 0.2,. 0.3, 0.4, 0.5, 0.6, 0.7, 0.8, 0.9, or 1.0) per dose of the composition is sufficient to induce at least 0.5 antitoxin units (A.U.) of anti-alpha toxin antibody per mL of antisera of an avian (e.g., chicken) vaccinated with the vaccine. In some embodiments, the composition can induce at least about 0.5, 1.0, 1.5, 2.0, 2.5, 3.0, 3.5, 4.0, 4.5, 5.0, 5.5, 6.0, 6.5, 7.0, 7.5. 8.0, 8.5, 9.0, 9.5 or 10 A.U. of anti-toxin antibody per mL of antisera of the avian.

As used herein an "antitoxin unit" or "A.U." of antitoxin antibody per mL of antiserum (which can be used interchangeably with "anti-Toxin Neutralizing Test" units or "TNT" units), is defined by the ability of sera to neutralize the toxic effects of a toxin in a mouse bioassay. In this test, a known amount of toxin, established by international standards as are known in the art, is mixed with serial dilutions of serum from vaccinated animals. The mixture is incubated one hour at room temperature and then injected intravenously into mice. The mice will survive if the toxin is completely neutralized by the sera, otherwise they die. The antitoxin units or titer is determined as the reciprocal of the highest dilution of sera that neutralized the toxin.

In further embodiments, the composition can comprise an antigen in a cell-free preparation. In other embodiments, the antigen can be an alpha toxoid in a *C*. *perfringens* alpha toxoid supernatant. In certain embodiments, the composition can comprise, consist essentially of and/or consist of an antigen that can be a *C*. *perfringens* Type A alpha toxoid and/or a *C*. *perfringens* Type C alpha toxoid. In some embodiments, the composition of this invention can comprise *C. perfringens* beta toxin, *C. perfringens* beta 2 toxin, *C*. *periringens* enterotoxin, *C*. *perfringens* epsilon toxin, *C*. *perfringens* iota toxin, *C*. *perfringen:s* kappa toxin, *C*. *perfringens* lambda toxin, *C*. *perfringens* theta toxin, *C. sordellii* hemorrhagic toxin, *C. sordellii* lethal toxin, *C. difficile* A toxin, *C*. *difficile* B toxin, *C*. *septicum* alpha toxin, *C*. *novyi* alpha toxin, *C*. *novyi* beta toxin and/or any combination thereof. Such a composition can further comprise, consist essentially of and/or consist of one or more viral antigens, one or more bacterial antigens, and/or one or more parasitic antigens as described herein.

The present invention is explained further in the following examples. In these Examples, ".µL" means microliters, ".µg" means micrograms, "mL" means milliliters, "cc" means cubic centimeters, "mm" means millimeters, "mM" means concentration in millimoles, "mg" means milligrams, "°C" means degrees Celsius and E18 and E19 mean embryonation days 18 and 19, respectively..

### EXAMPLES

**Example I.** Immune response following *in ovo* vaccination with commercially available *Clostridium perfringens* Type C&D vaccines (Siteguard G & Vision CD).

### Experimental Design

Broiler eggs were manually *in ovo* injected with commercially available *Clostridium perfringens* toxoid (Siteguard^{®} G) and bacterin-toxoid vaccines (Vision^{®} CD^{®} vaccine). Hatched birds were grown out to measure antibody responses. Site of Injection evaluation was performed. At day 0 (hatch) select treatment groups received a post hatch vaccination. All birds were housed in cages (5 birds/cage). Each cage was supplied with a diet of Normal Broiler Starter. On day 14 birds were switched over to Broiler Grower Feed. Birds were bled and serum was tested for an antibody response by serum-toxin neutralization assay.

### Materials & Methods

### Injection Material (Siteguard^{®} G):

Siteguard^{®} G (adjuvant unknown) is a *C*. *perfringens* type C & D toxoid vaccine produced by Schering-Plough. It protects sheep and cattle from diseases caused by type C & D toxins. For vaccination, 4.0 mL (cattle) or 2.0 mL (sheep) of the vaccine is administered subcutaneously (SQ) or intramuscularly (IM). Booster vaccinations are administered three to four weeks post initial vaccination and annually.

### Injection Material (Vision^{®} CD^{®}):

Vision^{®} CD^{®} (with the proprietary adjuvant 'Spur^{®}') is a *C*. *perfringens* type C & D bacterin-toxoid vaccine produced by Intervet. It protects cattle, sheep, and goats from enterotoxemia caused by *C*. *perfringens* type C & D. For vaccination, 2.0 mL of the vaccine is administered subcutaneously into the animal (cattle, sheep, or goat). Three to four weeks post initial vaccination, the animal receives an additional 2.0 mL (SQ) and is re-vaccinated annually thereafter.

### Injection Protocol:

On E19, broiler eggs were injected with test materials targeting either the amniotic fluid or embryo body of each egg. In addition, at day 0 post hatch, some *in ovo* and non-*in ovo* injected treatment groups received a vaccination or booster immunization of test materials. For the post-hatch vaccination or booster immunization, 0.5 mL of vaccine was administered by subcutaneous injection in the back of the neck.

### Site of Injection:

At the time of injection, eggs allotted for site of injection evaluation were injected with dye. Eggs were then euthanized and necropsied for site of injection evaluation. Site of injection was analyzed by likelihood ratio chi-square. (Table 1)

### Bleeding:

At days 7, 14, 21, and 28 post hatch, blood was collected from each individual bird and pooled in individual vacutainers (per treatment group). At days 7,14 and 21; ≤0.5 mL of blood was collected via either the wing or jugular. At day 28, ≤0.5 mL of blood was collected via cardiac puncture. Blood was then incubated at room temperature for one hour. Then the blood samples were placed in a table top centrifuge at 2400 RPMs for 10 minutes. Once centrifugation was complete, serum was removed from each blood sample and stored in a 96 well storage plate (2-8°C or -70°C) for future immune response evaluations.

### Clostridium perfringens Type C (beta) toxin Neutralization Testing in Mice: Sample Preparation for Mouse Inoculation

### A. Materials

1. *Clostridium perfringens* Type C (beta) toxin - CVBL Lot no. IRP513(04)
2. *Clostridium perfringens* Type C (beta) antitoxin - CVBL Lot no. IRP486 Receipt
3. Diluent - 1% peptone, 0.25% NaCI pH.7.2, BBL Lot no. 0510.06, NB ref. - NB 140 p. 87
4. Chicken Serum Samples - EMHE1381, NB 140 p. 80 a. Sample nos. 7, 8, 9, 10A, 10B, 11A, 11 B, 12A, 12B, 13A, 14A, 15A
5. Sterile 3 ml and 1.8 ml vials.

### B. Methods:

1. Dilute standard beta antitoxin 1:50 in diluent, 10 mls total.
   a. Thaw beta antitoxin at room temp.
   b. Mix 200 %µl of beta antitoxin and 9.8 ml of diluent = 1:50.
   c. Hold on ice.
2. Dilute beta toxin 1:120 in diluent, 12 mls total.
   a. Thaw beta toxin at room temp.
   b. Mix 200 µl of beta toxin and 1.8 ml of diluent = 1:10
   c. Mix 1 ml of the 1:10 diluted beta toxin with 11 mls of diluent = 1:120
   d. Hold on ice
3. Prepare the Lₒ control sample.
   a. Mix 0.5 ml of beta toxin (1:120 dilution) with 0.5 ml of diluent.
   b. Add 1 ml of beta antitoxin (1:50 dilution).
   c. Mix and incubate at room temperature for 1 hour.
   d. Hold samples on ice.
4. Prepare the L₊ control sample.
   a. Mix 0.8 ml of beta toxin (1:120 dilution) with 0.2 ml of diluent.
   b. Add 1 ml of beta antitoxin (1:50 dilution).
   c. Mix and incubate at room temperature for 1 hour.
   d. Hold samples on ice.
5. Prepare the 12 test serum samples.
   a. Mix 3.25 ml of beta toxin (1:120 dilution) with 3.25 ml of diluent.
   b. To each of the twelve 1.8 ml tubes, add 0.5 ml of toxin from step 5.a.
   c. Label each tube with sample number and treatment group number.
   d. Add 0.5 ml of each undiluted chicken serum to the appropriately labeled tube.
   e. Mix and incubate at room temperature for 1 hour.
   f. Store samples on ice.
6. All unused samples are stored at 2-7°C.

### Pre-Study Activities

Seventy-eight female Swiss white (CD-1) mice (16 -20 grams body weight) were purchased for use in the study. Mice were shipped from the vendor (Charles River Laboratories) and transported to the Clinical Testing Facility.

Mice were housed in cages placing 2 mice per cage for the chicken serum groups and 4 cages of 5 mice per cage for the control groups. The mice were held for an acclimation period of 5 days prior to initiation of the study on Day 0. Mice were housed and cared for according to standard operating procedures and fed a standard laboratory diet and offered water ad *libitum.*

### Day 0

Mice were examined for normal health and appearance and placed on test enrolling seventy-six mice. Two mice were not enrolled on study and were euthanized. Each mouse was injected intravenously (IV) with a 26g x 3/8 needle in the tail vein according to treatment groups described under STUDY DESIGN. Mice were monitored twice daily for signs of shock, pain or distress as evidenced by the following:
Lethargy
Huddling
Rough/ruffled hair coat
Hunched posture
Ataxia
Anorexia or inability to reach food and water
Moribund mice were euthanized via CO₂ overdose according to standard operating procedures.

### Day 1 (approximately 24 hours post inoculation)

Mice were observed and number of mortalities recorded.

### RESULTS

A positive specific antibody response was detected in sera from birds vaccinated *in ovo* (embryo body-targeted) with a commercial *C*. *perfringens* bacterin-toxoid vaccine with and without a post-hatch boost using the serum-toxin neutralization assay. These data indicate that administration of a *C*. *perfringens* bacterin-toxoid vaccine *in ovo* elicits partial protection and *in ovo* administration followed by a post-hatch boost confers full protection against *C*. *perfringens.* (Table 2)

### EXAMPLE II. Immune response following in ovo vaccination with an experimental vaccine preparation containing recombinant alpha toxin

### Experimental Design

The above study was conducted to determine if a humoral (antibody) immune response could be detected in broiler birds following *in ovo, in ovo +* post hatch or post hatch vaccinations with a *Clostridium perfringens* recombinant alpha toxin **(SEQ** ID **NO:6)** (provided by Dr. Glenn Songer, Dept. of Veterinary Science and Microbiology, The University of Arizona), adjuvanted with Incomplete Freund's Adjuvant and Quil-A. The immunization strategy included *in ovo* embryo body targeting at E18 as well as a day 7 post hatch vaccination. Antibody response was evaluated at 28 days of age.

On E18, Broiler eggs were manually *in ovo* injected with either control materials (Quil A; Accurate Chemical & Scientific Corporation, Product # AP04991, adjuvant grade, Batch # L77-238) emulsified with Incomplete Freund's Adjuvant (IFA; Rockland, Lot # 16235) or C. *perfringens* recombinant alpha toxin (55 or 60 µg/dose adjuvanted with Quil A + IFA (13 or 15 µg/dose). Site of injection evaluation was performed by injection of dye. At day 0 (hatch) birds were housed in cage units (5 birds/cage). Birds received Normal Broiler Starter. Additionally, some birds were vaccinated on day 7 or 17 according to treatment (0.2 mL of vaccine by subcutaneous injection in the back of the neck). Bird sera were then evaluated for specific antibody response via western blot.

### Materials & Methods

### Injection:

On E18, broiler eggs were injected with test materials (*Clostridium perfringens* alpha toxin + vaccine adjuvant) for targeting the embryo body of each egg. In addition, at day 7 or day 17 post hatch, some *in ovo* and non-*in ovo* injected treatment groups received a vaccination (Table 3)

### Site of Injection (SOI):

At the time of injection, eggs allotted for SOI evaluation were injected. with dye. Eggs were then euthanized and necropsied for SOI evaluation. (Table 4)

### Sera collection:

At days 7, 14, 21, and 28 post hatch, blood was collected from each individual bird and placed in individual vacutainers. At days 7, 14, and 21 ≤ 0.5mL of blood was collected via either the wing or jugular vein. At day 28, ≥5:0.5mL of blood was collected via cardiac puncture. Blood was then incubated at room temperature for 1 hour. Blood samples were centrifuged and serum was removed and stored in a 96 well storage plate (2-8°C or-70°C) for immune response evaluation.

### Western blot testing:

SDS slab gel electrophoresis was carried out according to the method of Laemmli (Nature 227:680-685 (1970)) as described by O'Farrell (J. Biol. Chem. 250:4007-4021 (1975), second dimension), using a 10% acrylamide slab gel (125 mm length X 150 mm width X 0.75 mm thickness) overlaid with a 25 mm stacking gel. Electrophoresis was carried out at 12 mAmp for about 3.5 hours or until the bromophenol blue front had migrated to the end of the slab gels. After slab gel electrophoresis, the gel for blotting was placed in transfer buffer (12.5 mM Tris, pH 8.8, 96 mM glycine, 20% MeOH) and transblotted onto a PVDF membrane overnight at 200 mA and approximately 100 volts/two gels. The PVDF membrane was then Coomassie blue stained and dried between sheets of filter paper.

The PVDF membrane was stained with Coomassie Brilliant Blue R-250 and desktop scanned before and after cutting into individual lanes. Each blot lane was placed in a separate container and blocked for two hours in 5% nonfat dry milk in Tween-20 Tris buffered saline (TTBS) and rinsed in TTBS. The blots were then incubated in primary antibody (diluted 1:100 in 2% nonfat dry milk in TTBS) overnight and rinsed 3 X 10 minutes in TTBS.

The blot lane 1 (positive control) was then placed in secondary antibody [rabbit anti-goat IgG-HRP (Sigma Cat. # A-5420 and Batch #034K4858), 1:5,000 diluted in 2% NFDM in TTBS] for two hours, rinsed 3 X 10 minutes in TTBS, treated with ECL and exposed to x-ray film.

The remaining blot lanes were then placed individually in secondary antibody [rabbit anti-chicken IgG-HRP (Bethyl Cat. # A30-107P and Batch # A30-107P-3), 1:2,000 diluted in 2% nonfat dry milk in TTBS] for two hours, rinsed 3 X 10 minutes in TTBS, treated with ECL and exposed to x-ray film.

Results of the western blotting studies are described in Table 5.

### Results

A specific antibody response was detected in birds vaccinated *in ovo* (embryo body-targeted) with recombinant alpha toxin adjuvanted with Quil-A & IFA with and without a post-hatch boost. These data demonstrate that *in* ovo vaccination with a recombinant alpha toxin can elicit an immune response against *C*. *perfringens* alpha toxin in broilers.

### EXAMPLE III.

A commercially available inactivated oil emulsion vaccine for Newcastle disease was purchased from Maine Biological Laboratories. The vaccine was administered *in ovo* on E18 via the amniotic fluid route or *in ovo* on E19 via the embryo body route. Site directed administration to the amniotic fluid and embryo body was confirmed by conducting a site of injection analysis using dye on E18 or E19. Site directed administration to the amniotic fluid was accomplished using a blunt needle (Group 2). Site directed administration to the embryo body was done using a sharp 1.25-inch needle (Group 3). Blood serum was collected at 14, 21 and 28 days of age and assayed for antibodies specific to Newcastle disease virus (NDV) using ELISA (idexx, Inc.). Different individual birds were bled on each blood collection day.

The E18 site of injection analysis indicated that 22/24 eggs were injected in the amniotic fluid, 22/24 in the allantoic fluid, and 0/24 in the embryo body. The E19 site of injection analysis indicated that 7/10 eggs were injected in the embryo body and 3/10 eggs were injected in the amniotic fluid. Table 6 shows the antibody response to Newcastle disease virus following *in ovo* vaccination of chickens. Table 7 shows percent hatch data.

The study demonstrates that the immune response of the developing embryo is strongly influenced by the *in ovo* administration site of inactivated oil emulsion Newcastle disease vaccine (Table 6). Embryos vaccinated in the amniotic fluid that surrounds the embryo body did not respond with Newcastle disease specific antibodies. On the other hand, embryos vaccinated directly into the body of the embryo responded with a strong antibody response that increased with age to 28 days. A total of 34 birds were bled over the course of this study and 26/34 were positive for antibodies to Newcastle disease virus (Table 6). 26/34 is 76.5%, which is very similar to the site of injection study where 70% of the embryos immunized on E19 were injected into the embryo body. Percent hatch was within normal ranges for treated and non-treated groups (Table 7).

### Example IV.

A commercially available inactivated oil emulsion vaccine for Newcastle disease was purchased from Maine Biological Laboratories. The vaccine was administered *in ovo* on E19 via the embryo body route or subcutaneous at hatch. Site directed administration to the embryo body was done using a sharp 1.25-inch needle (Group 3). Day of hatch vaccination was done by injecting vaccine subcutaneous in the nape of newly hatched chicks (Group 2). Blood serum was collected at 21 days of age and assayed for antibodies specific to NDV using ELISA (idexx, Inc.). The results are shown in Table 8.

The data presented in Table 8 show that embryos vaccinated in the body with the Newcastle disease oil emulsion vaccine responded as well as chicks vaccinated by the standard day of hatch route. Percent hatch was within normal ranges for treated and non-treated groups (Table 9).

The data presented in examples III and IV above show that hitting the embryo body is necessary to stimulate an active immune response to an inactivated antigen (in this case Newcastle disease virus). These data also indicate that embryos are not negatively affected by injection into the embryo body with an inactivated antigen in an oil-emulsion adjuvant.

*In ovo* (prior to hatch) embryo body injection may be accomplished manually using syringe and needle or by an automated injection device also using needles. In the examples given herein, syringe and needle were used manually to apply vaccine to the embryo body or the amniotic fluid (example III only) surrounding the embryo body. To accomplish the embryo body injection, the needle was inserted through a hole in the shell at the air cell end of the egg. The inserted needle passed through the air cell membrane, the allantoic membranes and fluid and finally into the amnion cavity where the embryo body resides. Next the needle penetrated the embryo body and vaccine was deposited. Embryo body injections can occur in numerous sites within the embryo's body and include subcutaneous, intra-dermal, intravenous, intramuscular and intra-abdominal deposition of vaccine, as well as any combination of these sites. Furthermore, embryo body injections can occur in the head, neck, shoulder, wing, back, breast or leg, including any combinations. Embryo body injection does not include exclusive vaccine deposition in the air cell, the allantoic cavity, the amniotic fluid or the albumin.

Embryo body injection *in ovo* may be done using needle of a length ranging from % inch to up 4 inches and gauges ranging from 15 to 28. Needle tips may range from very sharp (hypodermic) to blunt.

In examples III and IV, Newcastle disease virus vaccine was used as the model antigen. However, any properly formulated oil emulsion vaccine with enough antigenic mass would be expected to be similar to the Newcastle disease vaccine tested. Therefore, inactivated vaccines to infectious bursal disease, avian influenza, infectious bronchitis, chick infectious anemia virus, laryngotracheitis, avian reovirus, adenovirus, rotavirus, astrovirus, inclusion body hepatitis, egg drop syndrome, *Escherichia coli, Mycoplasma* spp., *Salmonella* spp., *Campylobacter* spp, *Clostridium* spp., *Haemophilus* spp, *Pasteurella* spp. can be delivered *in ovo* directly to the embryo's body according to the methods described herein. Vaccines made from these agents may be whole cell or subunit. Vaccines made from these agents may be produced conventionally in growth media, eggs or tissue culture and/or may be produced by recombinant means according to methods well known in the art. In addition, any disease agent that can be produced to have enough antigenic mass to effectively vaccinate day of hatch chicks when inactivated would also be expected to effectively vaccinate embryos *in ovo* if delivered directly to the embryo body.

The adjuvant used in the vaccine tested in these examples was a typical commercial oil emulsion. Non-oil emulsion inactivated vaccines with adjuvants other than oil would be expected to produce an active immune response if delivered directly to the embryo body prior to hatch. Adjuvants suitable.would include, but are not limited to, mineral gels, polyanions, pluronic polyols, saponin derivatives, lysolecithin and other similar surface active substances, glycosides and all types of oils and combinations thereof.

### EXAMPLE V.

Specific pathogen free (SPF) leghorns were vaccinated *in ovo* as follows Group 1: phosphate buffered saline (PBS); Groups 2 and 3: 0.3 x 10⁹ inactivated NDV EID₅₀/dose in PBS; Group 4: 0.3 x 10⁹ inactivated NDV EID₅₀/dose mixed with an alum depot adjuvant (Imject, Pierce; aluminum hydroxide and magnesium hydroxide); Group 5: a commercial oil emulsion vaccine for NDV. On day 11 of age, Group 3 subjects received a second dose of NDV in PBS by subcutaneous injection. The vaccines given *in ovo* were targeted to the embryo body and a site of injection analysis using dye was conducted on a separate set of like eggs to estimate the percent of embryos injected directly into the embryo body. The *in ovo* vaccination was done on day 19 of incubation with a 23 gauge 1.25 inch needle. Fourteen birds per group were placed in cages and grown to 21 days of age.

Serum samples were collected on day 21 of age and tested for IgG antibody to NDV by ELISA (Idexx, Inc.). Serum samples from Groups 2, 4 and 5 were also tested for NDV specific antibody by hemagglutination inhibition (HI) using four HA units. The number of samples tested by HI differs from those tested by ELISA because with several samples there was not enough serum collected to conduct the HI test. Birds were considered to have shown a measurable antibody response (i.e., seroconverted) to the vaccination if the serum sample had an ELISA titer ≥ 200 or an HI titer of ≥ 3.0 log₂ (i.e. titer of 1:8).

### Results.

The site of injection analysis indicates that embryo body injections accounted for 78% of all injections (Table 10).

The percent hatch and rate of seroconversion to NDV as measured by ELISA are shown in Table 11. In Table 12, the number of birds that seroconverted using the HI test is reported.

From these studies, the following key points were noted. 1) NDV antigen in PBS did not stimulate a measurable antibody response by NDV ELISA, even when the inactivated NDV antigen was given twice as in Group 3 (once *in ovo* and again on day 11 of age); 2) The NDV-Alum (Group 4) stimulated seroconversion in 8/14 birds when measured by ELISA, while the commercial oil emulsion vaccine (Group 5) stimulated seroconversion in 10/13 birds when measured by ELISA; 3) The NDV-Alum (Group 4) stimulated seroconversion in 12/14 when measured by HI, while the oil emulsion vaccine stimulated seroconversion in 11/11 birds when measured by HI; and 5) The commercial oil emulsion vaccine for Newcastle disease (Group 5) stimulated a stronger antibody response than did the NDV-Alum vaccine (Group 4). Example III shows that *in ovo* administration of an antigen presented in an oil emulsion depot adjuvant required the vaccine to be delivered to the embryo body to stimulate a measurable antibody response by ELISA. In the present example, *in ovo* site of injection analysis indicated that 78% of eggs received vaccine directly in the embryo body.

### EXAMPLE VI.

A study was conducted using SPF leghorns to determine if alum depot adjuvant stimulated an immune response when administered *in ovo.* The groups tested were as follows: Group 1: phosphate buffered saline (PBS) in ovo; Groups 2 and 3: 1.2 x 10⁹ EID₅₀ β-propiolactone inactivated NDV/dose in PBS in ovo; Group 4: *in ovo* administration of 1.2 x 10⁹ EID₅₀ β-propiolactone inactivated NDV/dose mixed with alum at a 30%:70% alum to NDV antigen ratio. The alum used was a commercial solution of aluminum hydroxide and magnesium hydroxide (Imject, Peirce). Group 3 received an additional dose of NDV antigen in PBS on day 11 of age by subcutaneous injection. The *in ovo* vaccine administration was done on day 19 of incubation using a 23 gauge 1.25 inch needle. The vaccines were targeted *in ovo* to the embryo body and a site of injection analysis was conducted on a separate set of like eggs to estimate the percent of embryos injected directly into the embryo body. Serum samples were taken on day 21 of age and tested for antibody to NDV by ELISA (Idexx, Inc.). Birds were considered to have shown a measurable antibody response (i.e., seroconverted) if the ELISA had a titer ≥ 200.

### Results.

The site of injection analysis indicates that 81 % of embryos were injected directly into the embryo body (Table 13). The percent hatch and rate and magnitude of seroconversion against Newcastle diseases virus are shown in Table 14.

These studies provided the following key points: 1) Embryos were injected on E19 and the site of injection data indicated that 81% of embryos were injected in the body of the embryo; and 2) In this study, the alum was mixed with β-propiolactone inactivated NDV in a 30% to 70% ratio and this differed from the study described herein in Example V, in which heat inactivated NDV was mixed at a 50% alum to 50% NDV antigen ratio. The difference in immune responses in the two studies may be due to the differences in NDV antigen used and/or the difference in alum to antigen ratios tested as well as differences in the ELISA used to measure the antibodies.

In example III it was shown that antigen presented in the depot adjuvant oil emulsion requires the vaccine to be delivered to the embryo body *in ovo* to stimulate a measurable antibody response by ELISA. In this study the site of injection analysis indicated that 81 % of embryos were injected in the embryo body, and from these data it would be expected that approximately 11 of the 14 eggs vaccinated *in ovo* would respond with an antibody response. The actual number that responded was nine of 14.

### Example VII.

A commercial oil emulsion Newcastle disease vaccine was given via the *in ovo* route to broilers. This study determined the ability of broilers to respond to inactivated Newcastle disease virus antigens when delivered in ovo by the amniotic fluid route and the intra-embryo route. Birds were bled at 13, 21, 26 and 35 days of age and antibody titer to NDV was determined using ELISA (Idexx, Inc.). Site of injection analysis was conducted on E18 and E19 using dye.

Hatch data are shown in Table 16. Percent hatch was normal when the oil emulsion vaccine was delivered into the embryo body.

Site of injection (Table 15) was very accurate with greater than 90% of embryos injected by the route indicated for the treatment (Table 16).

The Newcastle disease virus specific antibody response data are shown in Table 17. It can be seen that birds responded to the Newcastle antigen when the vaccine was delivered *in ovo* into the embryo body or subcutaneous at hatch. When the NDV vaccine was delivered in ovo to the amniotic fluid, there was no antibody response, indicating that the vaccine has to be given to the embryo body to stimulate an appropriate immune response.

The invention is defined by the following claims.

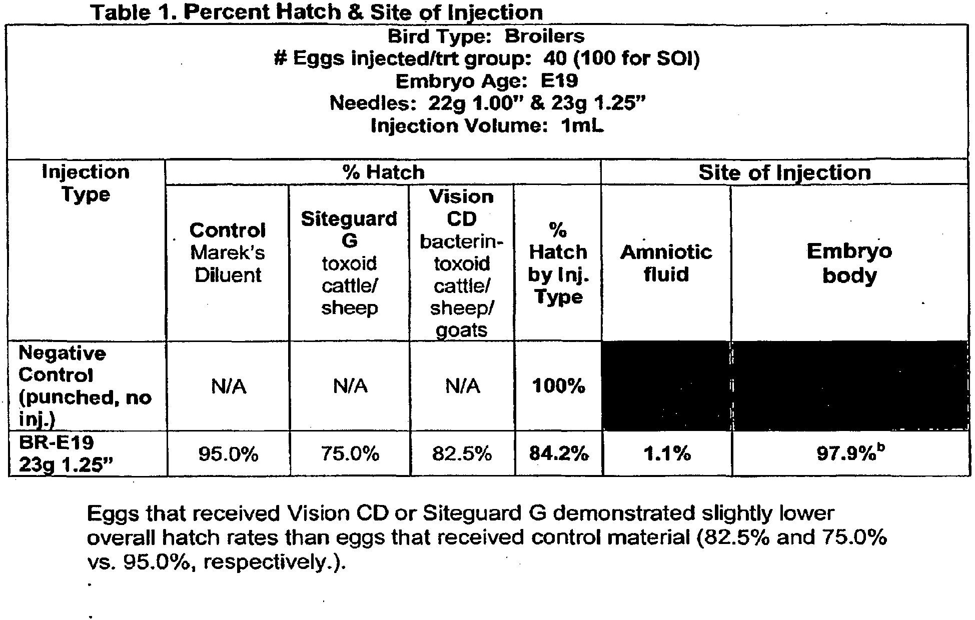

**Table 2. Serum-toxin neutralization assay results:**

| Study # | Bird type | Dose | Trt | Description | # healthy mice | Result |
|---|---|---|---|---|---|---|
| 1 | SPF | -- | -- | D0 SPF sera NEG CTL | 0/2 | **Valid negative control.** Neither mouse survived, as expected, indicating specific Abs were not present. |
| 2 | SPF | 1mL | 4 | D49 sera Hyperimmunized POS CTL (vaccination + 2 boosts) | 2/2 | **Valid positive control.** Both mice survived as expected, indicating specific antibodies were present. |
| 3 | BR | 1mL | 7 | D28 sera toxoid (Siteguard G) - EMBRYO BODY IN OVO | 0/2 | **No protection** |
| | | 1 mL | 8 | D28 toxoid (Siteguard G) - EMBRYO BODY IN OVO + post-hatch boost | 0/2 | **No protection** |
| | | 1 mL | 11 | D28 sera bacterin-toxoid (Vision CD) - EMBRYO BODY IN OVO | 1/2 1/2 | **Partial protection** |
| | | 1 mL | 12 | D28 sera bacterin-toxoid (Vision CD) - EMBRYO BODY IN OVO + post-hatch boost | 2/2 | **Complete protection - Satisfactory** |

| | | | | | | |
|---|---|---|---|---|---|---|
| All sera were pooled (~5 birds/group) • A specific positive response was detected in birds vaccinated *in ovo* (embryo body-targeted) with the *C*. *perfringens* bacterin-toxoid vaccine (Vision CD) followed by a post-hatch boost. Results suggested a low antibody response (partial protection) in birds vaccinated *in ovo* alone (no post-hatch boost). • No protection was observed in sera from birds vaccinated with a *C*. *perfringens* toxoid vaccine (Siteguard). | | | | | | |

**Table 3. Study Summary**

| | **Bird** | Broiler | |
|---|---|---|---|
| | **Day of Injection** | E18, D7, and D17 | |
| | **Type of Injections** | Embryo +/- Post Hatch boost (D7), Post hatch only (D7) w/ boost (D17) | |
| | **Control** | Non-injected (punched) | |
| | **Test Materials** | Inactivated Recombinant Alpha Toxin (SEQ ID NO:6), adjuvanted with Quil A + Incomplete Freund's Adjuvant | |
| | **Toxin Dose** | 55.2-60 µg/0.2 mL | |

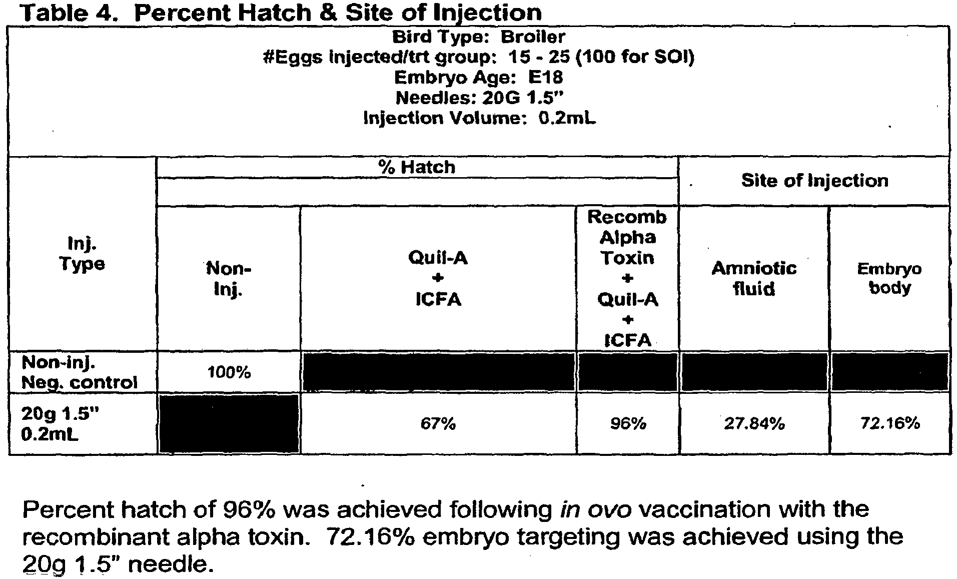

**Table 5. Detection of specific antibody response via western blot. Lane Sample ID Description Result**

| **Lane** | **Sample ID** | **Description** | **Result** |
|---|---|---|---|
| 1 | Positive control - goat anti alpha | **Positive control** pooled sera | **+** |
| 2 | Negative control - D0 SPF | **Negative Control** RP-967 pooled sera | **-** |
| D28 sera | | | |
| 3 | #1 | Trt 4A - ***in ovo* negative vehicle control** Pooled sera from 3 birds | **-** |
| 4 | #2 | Trt 5A - ***in ovo* embryo-vaccinated** +Pooled sera from 3 birds | **+** |
| 5 | #3 | Trt 5A - ***in ovo* embryo-vaccinated** Pooled sera from 3 birds | **+** |
| 6 | #4 | Trt 6A - ***in ovo* embryo + post-hatch vacc** Pooled sera from 3 birds | **+** |
| 7 | #5 | Trt 6A - ***in ovo* embryo + post-hatchvacc** Pooled sera from 3 birds | **+** |
| 8 | #6 | Trt 2A - **post-hatch positive control** Pooled sera from 3 birds | **+** |

| | | | |
|---|---|---|---|
| • Positive and negative controls performed as expected, demonstrating test validity. • A specific antibody response was detected in birds vaccinated *in ovo* (embryo body-targeted) with the recombinant alpha toxin formulation with and without a post-hatch boost. | | | |

**Table 6. Antibody response of chickens to Newcastle diseases virus following site directed in ovo administration of an inactivated oil emulsion Newcastle disease vaccine**

| GP | Vaccine Route | Day 14 of age | | Day 21 of age | | Day 28 of age | |
|---|---|---|---|---|---|---|---|
| | | Mean titer | # pos./ # tested¹ | Mean titer | # pos./ # tested | Mean titer | # pos./ # tested |
| 1 | Non-vaccinated | 7 | 0/10 | 1 | 0/12 | 23 | 0/12 |
| 2 | NDV vaccine in ovo amniotic fluid | 1 | 0/10 | 1 | 0/12 | 1 | 0/12 |
| 3 | NDV vaccine in ovo Embryo body | 909 | 7/10 | 3262 | 8/12 | 7819 | v11/12 |
| ¹ number of birds positive for antibodies to Newcastle disease/number of birds tested. | | | | | | | |

**Table 7: Percent hatch following site directed in ovo administration of an inactivated oil emulsion Newcastle disease vaccine**

| Group | Vaccine | Percent hatch data | |
|---|---|---|---|
| | Route | # hatched/# transferred | Percent hatched |
| 1 | Non-vaccinated | 48/50 | 96% |
| 2 | NDV vaccine in ovo amniotic fluid | 51/60 | 85% |
| 3 | NDV vaccine in ovo Embryo body | 48/60 | 80% |
| | | | |

**Table 8: Antibody response of chickens to Newcastle disease virus following intra-embryo in ovo administration or subcutaneous administration at day of hatch of an inactivated oil emulsion Newcastle disease vaccine**

| Group | Vaccine | Day 21 of age | |
|---|---|---|---|
| | Route | Mean titer | # pos./# tested¹ |
| 1 | Buffer in ovo Embryo body | 1 | 0/12 |
| 2 | NDV vaccine Subcutaneous at hatch | 3785 | 10/12 |
| 3 | NDV vaccine in ovo Embryo body | 3261 | 12/12 |
| ¹ number of birds positive for antibodies to Newcastle disease/number of birds tested | | | |

**Table 9: Percent hatch following intra-embryo in ovo administration of an inactivated oil emulsion Newcastle disease vaccine**

| Group | Vaccine | Percent hatch data | |
|---|---|---|---|
| | Route | # hatched/# transferred | Percent hatched |
| 1 | Buffer in ovo Embryo body | 17/21 | 81% |
| 2 | Non-vaccinated | 80/101 | 79.2% |
| 3 | NDV vaccine in ovo Embryo body | 18/20 | 90% |
| | | | |

**Table 10: Site of Injection using dye**

| **Air Cell** | **Allantoic sac** | **Amniotic fluid** | **Embryo Body** | **Yolk Sac** | **N** |
|---|---|---|---|---|---|
| 0% | 0% | 22.0% | 78.0% | 0% | 50 |

**Table 11: Percent hatch and NDV ELISA results of birds vaccinated in ovo with NDV antigen, NDV antigen-Alum or an oil emulsion NDV vaccine**

| **Group** | **Vaccine** | **Day 11 Boost** | **Hatch of Total (%)** | **Number of birds seroconverted/Total tested** | **Mean titer of birds that seroconverted by ELISA (day 21 of age)** |
|---|---|---|---|---|---|
| 1 | None | No | 96 | 0/13 | n/a |
| 2 | NDV | No | 77 | 0/14 | n/a |
| 3 | NDV | Yes | 86 | 0/14 | n/a |
| 4 | NDV-Alum | No | 88 | 8/14 | 1272 |
| 5 | NDV-OE** | No | 87 | 10/13 | 3038 |

| | | | | | |
|---|---|---|---|---|---|
| ** NDV-OE = commercial oil emulsion vaccine for NDV (LAHI) n/a = not applicable | | | | | |

**Table 12: NDV hemagglutination-inhibition results of broilers vaccinated in ovo with NDV antigen-Alum or an oil emulsion NDV vaccine**

| **Group** | **Treatment** | **HI titer (log₂) Mean ± SD** | **Number of birds HI Titer ≥3/ number of birds tested (day 21 of age)** |
|---|---|---|---|
| 2 | NDV | 1.8 ± 0.4 | 0/13 |
| 4 | NDV-Alum | 3.8 ± 1.1 | 12/14 |
| 5 | NDV-OE** | 8.9 ± 2.2 | 11/11 |

| | | | |
|---|---|---|---|
| ** NDV-OE = commercial oil emulsion vaccine for NDV (LAHI) | | | |

**Table 13: Site of Injection Results**

| **Treatment** | **Air Cell** | **Allantoic sac** | **Amniotic fluid** | **Embryo body** | **Yolk Sac** | **N** |
|---|---|---|---|---|---|---|
| **23G x 1.25"** | 0% | 2.1% | 16.7% | 81.3% | 0% | 48 |

**Table 14: Percent hatch and ELISA Results of broiler chickens vaccinated in ovo with NDV antigen or NDV-Alu,**

| **Group number** | **Antigen-Adjuvant** | **Day 11 Boost** | **Hatch of Total (%)** | **Number of birds seroconverted/Total tested** | **Mean titer of birds that seroconverted by ELISA (day 21 of age)** |
|---|---|---|---|---|---|
| 1 | None | No | 97 | 0/11 | n/a |
| 2 | NDV | No | 94* | 0/14 | n/a |
| 3 | NDV | Yes | | 1/14 | 200 |
| 4 | NDV-Alum | No | 96 | 9/14 | 411 |

| | | | | | |
|---|---|---|---|---|---|
| *Both NDV treatments were from the same group of hatched birds; n/a = not applicable | | | | | |

**Table 15: site of injection for site directed in ovo delivery of an inactivated Newcastle disease virus oil emulsion vaccine.**

| Embryo age at injection | | Site of injection | | |
|---|---|---|---|---|
| Day 18.0 | | Embryo body | Amniotic fluid | Allantoic fluid |
| | | 1/34 | 33/34 | 0 |
| | % | 2.9 | 97.1 | 0 |
| Day 19 | | Embryo body | Amniotic fluid | Allantoic fluid |
| | | 57/63 | 6/63 | 0 |
| | % | 90.5 | 9.5 | 0.0 |

**Table 16: Treatment groups and percent hatch for groups of broilers given an oil emulsion NDV vaccine in ovo.**

| **Gp #** | **Description¹** | **Dose (ml)** | **Route** | **# injected/# hatch** | **Percent hatch** |
|---|---|---|---|---|---|
| 1 | Non-vaccinated | NA | NA | 39/40 | 97.5 |
| 2 | NDV oil emulsion | 0.1 | Amniotic fluid in ovo | 16/21 | 76.2 |
| 3 | NDV oil emulsion | 0.1 | embryo body in ovo | 21/21 | 100.0 |
| 4 | NDV oil emulsion | 0.1 | subcutaneous at hatch | 20/20 | 100.0 |

| | | | | | |
|---|---|---|---|---|---|
| ¹ NDV oil emulsion - formulated for day of age chicks, 1 dose in 0.1 ml | | | | | |

**Table 17: Antibody response to NDV in broilers immunized with an NDV oil emulsion vaccine in ovo.**

| **Gp #** | **Description¹** | **Route** | **Antibody titer to NDV on day of age** | | | |
|---|---|---|---|---|---|---|
| | | | **13** | **21** | **26** | **35** |
| 1 | Non-vaccinated | NA | 14 | 10 | 24 | 14 |
| 2 | NDV oil emulsion | Amniotic fluid in ovo | 5 | 8 | 23 | 34 |
| 3 | NDV oil emulsion | embryo body in ovo | 14 | 1646 | 1645 | 2370 |
| 4 | NDV oil emulsion | subcutaneous at hatch | 16 | 1057 | 1346 | 1852 |

| | | | | | | |
|---|---|---|---|---|---|---|
| ¹ NDV oil emulsion - formulated for day of age chicks, 1 dose in 0.1 ml | | | | | | |

## Claims

1. An immunogenic composition for use in inducing an immune response against a *Clostridium* species, wherein the immunogenic composition comprises an inactivated *Clostridium* toxin (toxoid) and/or an inactivated *Clostridium* bacterium (bacterin), and wherein said composition is administered by *in ovo* injection directly into the embryo body of an avian subject.

2. The immunogenic composition for use of claim 1, wherein the immunogenic composition is administered to the embryo parenterally.

3. The immunogenic composition for use of claim 1, wherein the avian subject is a chicken.

4. The immunogenic composition for use of claim 3, wherein the immunogenic composition is administered from day 15 to day 20 of incubation.

5. The immunogenic composition for use of claim 3, wherein the immunogenic composition is administered on day 18 or 19 of incubation.

6. The immunogenic composition for use of claim 1, wherein the avian subject is a turkey.

7. The immunogenic composition for use of claim 1, wherein the inactivated *Clostridium* toxin (toxoid) and/or bacterium (bacterin) is/are an inactivated *Clostridium perfringens* toxin (toxoid) and/or bacterium (bacterin).

8. The immunogenic composition for use of claim 1, wherein the inactivated *Clostridium* toxin (toxoid) is a *Clostridium* alpha toxin (toxoid).

9. The immunogenic composition for use of claim 8, wherein the inactivated *Clostridium* alpha toxin (toxoid) is *Clostridium perfringens alpha* toxin (toxoid).

10. The immunogenic composition for use of claim 1, wherein the inactivated *Clostridium* toxin (toxoid) is recombinant.

11. The immunogenic composition for use of claim 1, wherein the immunogenic composition comprises a water-in-oil-in-water emulsion.

12. The immunogenic composition for use of claim 1, wherein the immunogenic composition comprises an adjuvant.

13. The immunogenic composition for use of claim 12, wherein the adjuvant comprises an aluminium-derived adjuvant, a saponin, an oil, or any combination of the foregoing.

14. The immunogenic composition for use of claim 1, further comprising (a) administering *in ovo* an immune stimulant at any time during incubation, (b) administering *in ovo* a coccidiosis vaccine, a Marek's disease vaccine, an infectious bursal disease vaccine, a Newcastle disease vaccine, a fowl pox vaccine, or any combination of the foregoing, or (c) administering *in ovo* a nutrient formulation, an enteric modulator, or a combination thereof.

## Patentansprüche

1. Immunogene Zusammensetzung zur Verwendung beim Induzieren einer Immunantwort gegen eine *Clostridium-Spezies,* wobei die immunogene Zusammensetzung ein inaktiviertes *Clostridium-*Toxin (Toxoid) und/oder ein inaktiviertes *Clostridium-*Bakterium (Bakterin) umfasst und wobei die Zusammensetzung durch *In-ovo*-Injektion direkt in den Embryokörper eines aviären Subjektes verabreicht wird.

2. Immunogene Zusammensetzung zur Verwendung nach Anspruch 1, wobei die immunogene Zusammensetzung parenteral an den Embryo verabreicht wird.

3. Immunogene Zusammensetzung zur Verwendung nach Anspruch 1, wobei das aviäre Subjekt ein Huhn ist.

4. Immunogene Zusammensetzung zur Verwendung nach Anspruch 3, wobei die immunogene Zusammensetzung an Tag 15 bis Tag 20 der Inkubation verabreicht wird.

5. Immunogene Zusammensetzung zur Verwendung nach Anspruch 3, wobei die immunogene Zusammensetzung von Tag 18 oder 19 der Inkubation verabreicht wird.

6. Immunogene Zusammensetzung zur Verwendung nach Anspruch 1, wobei das aviäre Subjekt ein Truthuhn ist.

7. Immunogene Zusammensetzung zur Verwendung nach Anspruch 1, wobei das inaktivierte *Clostridium-Toxin* (Toxoid) und/oder Bakterium (Bakterin) ein inaktiviertes *Clostridium perfringens*-Toxin (Toxoid) und/oder -Bakterium (Bakterin) sind/ist.

8. Immunogene Zusammensetzung zur Verwendung nach Anspruch 1, wobei das inaktivierte *Clostridium*-Toxin (Toxoid) ein *Clostridium-*alpha-Toxin (Toxoid) ist.

9. Immunogene Zusammensetzung zur Verwendung nach Anspruch 8, wobei das inaktivierte *Clostridium*-alpha-Toxin (Toxoid) *Clostridium-perfringens-*alpha-Toxin (Toxoid) ist.

10. Immunogene Zusammensetzung zur Verwendung nach Anspruch 1, wobei das inaktivierte *Clostridium*-Toxin (Toxoid) rekombinant ist.

11. Immunogene Zusammensetzung zur Verwendung nach Anspruch 1, wobei die immunogene Zusammensetzung eine Wasser-in-Öl-in-Wasser-Emulsion umfasst.

12. Immunogene Zusammensetzung zur Verwendung nach Anspruch 1, wobei die immunogene Zusammensetzung ein Adjuvans umfasst.

13. Immunogene Zusammensetzung zur Verwendung nach Anspruch 12, wobei das Adjuvans ein von Aluminium abgeleitetes Adjuvans, ein Saponin, ein Öl oder eine beliebige Kombination der vorstehenden umfasst.

14. Immunogene Zusammensetzung zur Verwendung nach Anspruch 1, außerdem umfassend: (a) Verabreichen *in ovo* eines Immunstimulans zu einer beliebigen Zeit während der Inkubation, (b) Verabreichen *in ovo* eines Kokzidiose-Impfstoffs, eines Impfstoffs gegen die Marek'sche Krankheit, eines Impfstoffs gegen infektiöse Bursitis, eines Impfstoffs gegen Newcastle-Krankheit, eines Impfstoffs gegen Geflügelpocken oder einer beliebigen Kombination der vorstehenden oder (c) Verabreichen *in ovo* einer Nährstoffformulierung, eines enterischen Modulators oder einer Kombination davon.

## Revendications

1. Composition immunogène pour son utilisation dans l'induction d'une réponse immunitaire dirigée contre une espèce de *Clostridium,* dans laquelle la composition immunogène comprend une toxine inactivée de *Clostridium* (anatoxine) et/ou une bactérie *Clostridium* inactivée (bactérine), et dans laquelle ladite composition est administrée par injection *in ovo* directement dans le corps embryonnaire d'un sujet aviaire.

2. Composition immunogène pour son utilisation selon la revendication 1, dans laquelle la composition immunogène est administrée par voie parentérale à l'embryon.

3. Composition immunogène pour son utilisation selon la revendication 1, dans laquelle le sujet aviaire est un poulet.

4. Composition immunogène pour son utilisation selon la revendication 3, dans laquelle la composition immunogène est administrée à partir du jour 15 au jour 20 de l'incubation.

5. Composition immunogène pour son utilisation selon la revendication 3, dans laquelle la composition immunogène est administrée au jour 18 ou 19 de l'incubation.

6. Composition immunogène pour son utilisation selon la revendication 1, dans laquelle le sujet aviaire est un dindon.

7. Composition immunogène pour son utilisation selon la revendication 1, dans laquelle la toxine inactivée de *Clostridium* (anatoxine) et/ou la bactérie *Clostridium* inactivée (bactérine) est/sont une toxine inactivée de *Clostridium perfringens* (anatoxine) et/ou une bactérie *Clostridium perfringens* inactivée (bactérine).

8. Composition immunogène pour son utilisation selon la revendication 1, dans laquelle la toxine inactivée de *Clostridium* (anatoxine) est une toxine alpha de *Clostridium* (anatoxine).

9. Composition immunogène pour son utilisation selon la revendication 8, dans laquelle la toxine alpha inactivée de *Clostridium* (anatoxine) est une toxine alpha de *Clostridium perfringens* (anatoxine).

10. Composition immunogène pour son utilisation selon la revendication 1, dans laquelle la toxine inactivée de *Clostridium* (anatoxine) est recombinante.

11. Composition immunogène pour son utilisation selon la revendication 1, dans laquelle la composition immunogène comprend une émulsion eau dans l'huile dans l'eau.

12. Composition immunogène pour son utilisation selon la revendication 1, dans laquelle la composition immunogène comprend un adjuvant.

13. Composition immunogène pour son utilisation selon la revendication 12, dans laquelle l'adjuvant comprend un adjuvant dérivé de l'aluminium, une saponine, une huile ou n'importe quelle combinaison des éléments susmentionnés.

14. Composition immunogène pour son utilisation selon la revendication 1, comprenant en outre
(a) l'administration *in ovo* d'un stimulant de l'immunité à n'importe quel moment pendant l'incubation,
(b) l'administration *in ovo* d'un vaccin contre la coccidiose, d'un vaccin contre la maladie de Marek, d'un vaccin contre la bursite infectieuse, d'un vaccin contre la maladie de Newcastle, d'un vaccin contre la variole aviaire ou de n'importe quelle combinaison des vaccins susmentionnés, ou (c) l'administration *in ovo* d'une formulation de nutriments, d'un modulateur entérique ou d'une combinaison de ceux-ci.
